(19) Europäisches Patentamt
European Patent Office
Office européen des brevets

(11) EP 3 421 608 B1

(12) EUROPEAN PATENT SPECIFICATION

(45) Date of publication and mention of the grant of the patent:
**09.03.2022 Bulletin 2022/10**

(21) Application number: **17756179.2**

(22) Date of filing: **07.02.2017**

(51) International Patent Classification (IPC):
***C12Q 1/68*** *(2018.01)* ***C12N 15/09*** *(2006.01)*

(52) Cooperative Patent Classification (CPC):
**G16B 30/10; C12Q 1/68; G16B 30/00;** C12N 15/09

(86) International application number:
**PCT/JP2017/004390**

(87) International publication number:
**WO 2017/145738 (31.08.2017 Gazette 2017/35)**

# (54) CHROMOSOME NUMBER QUANTIFICATION METHOD

QUANTIFIZIERUNGSVERFAHREN FÜR CHROMOSOMENANZAHL

PROCÉDÉ DE QUANTIFICATION DU NOMBRE DE CHROMOSOMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.02.2016 JP 2016033284**

(43) Date of publication of application:
**02.01.2019 Bulletin 2019/01**

(73) Proprietor: **FUJIFILM Corporation**
**Tokyo 106-8620 (JP)**

(72) Inventor: **TSUJIMOTO Takayuki**
**Ashigara-kami-gun**
**Kanagawa 258-8538 (JP)**

(74) Representative: **Klunker IP**
**Patentanwälte PartG mbB**
**Destouchesstraße 68**
**80796 München (DE)**

(56) References cited:
**WO-A1-2008/004691 US-A1- 2010 070 452**
**US-A1- 2013 123 120**

- **ZIMMERMANN B ET AL: "P-448 One day turn around 24-chromosome preimplantation genetic diagnosis by targeted sequencing enabled by multiplex PCR and fast benchtop sequencers", HUMAN REPRODUCTION, OXFORD JOURNALS, GB, vol. 27, no. Suppl. 2, 1 January 2012 (2012-01-01), pages P-448, XP009508402, ISSN: 0268-1161, DOI: 10.1093/HUMREP/27.S2.87**
- **ZIMMERMANN, B. ET AL.: 'P-448 One day turn around 24-chromosome preimplantation genetic diagnosis by targeted sequencing enabled by multiplex PCR and fast benchtop sequencers' HUM. REPROD. vol. 27, no. 2, 2012, page P-448, XP009508402**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 421 608 B1

## Description

## BACKGROUND OF THE INVENTION

1. Field of the Invention

The present invention relates to a chromosome number determination method comprising a particular method for designing primer sets.

2. Description of the Related Art

Genetic analysis such as deoxyribonucleic acid (DNA) base sequence analysis can be easily performed using a next generation sequencer or the like which has been developed recently. However, the total base length of a genome is generally enormous. On the other hand, there is a restriction on the reading ability of the sequencer. Therefore, in general, only a specific gene region required is amplified to limitedly read base sequences thereof. A polymerase chain reaction (PCR) method has been widespread as a technique for efficiently and precisely amplifying only a specific gene region required. Particularly, a technique for selectively amplifying a plurality of gene regions by simultaneously supplying a plurality of types of primers to one PCR reaction system is called multiplex PCR.

However, since it is difficult to directly perform PCR on a small amount of DNA such as a single cell, a region of interest is enriched through multiplex PCR and/or hybridization after amplification of the whole genome region using whole genome amplification (WGA). However, since WGA has a large amplification bias, it is difficult to accurately perform quantitative determination of the number of chromosomes.

WO2014/018080A discloses a method for reducing production of non-target amplification products generated through multiplex PCR and simultaneously amplifying a large number (one thousand to several tens of thousands) of genes to quantitatively determine chromosomes or the like. More specifically, in a case where primers are designed, an "undesirability score" between primers is designed to be less than a threshold value, and the "undesirability score" is designed so that the likelihood of formation of a primer dimer (dimer of primer) is less than or equal to a threshold value. However, there is no description of a method for specifically calculating the "undesirability score", and it is considered that it is impossible to avoid generation of a primer dimer.

In addition, a method for designing a primer for multiplex PCR which can efficiently amplify a plurality of amplification sites (targets) is disclosed in WO2008/004691A and US 2010/070452 A1, respectively.

ZIMMERMANN B ET AL: "P-448 One day turn around 24-chromosome preimplantation genetic diagnosis by targeted sequencing enabled by multiplex PCR and fast benchtop sequencers", HUMAN REPRODUCTION, OXFORD JOURNALS, GB, vol. 27, no. Suppl. 2, 1 January 2012 (2012-01-01), pages P-448, discloses multiplex PCR performed using single cells derived from trisomy patients for pre-implantation diagnosis. It is disclosed that at least 500 loci per chromosome were analyzed, and that analysis of approximately 100 loci per chromosome can enable diagnosis with an accuracy of 99% or better.

## SUMMARY OF THE INVENTION

In order to improve sensitivity of the multiplex PCR itself and accurately amplify a small amount of DNA, it is conceivable to increase the number of amplified loci and increase the amount of data to be acquired. In general, however, an increase in the number of primer sets used in multiplex PCR causes formation of primer dimers and an increase in nonspecific amplification products such as amplification products from a region of non-interest. For this reason, in general multiplex PCR, even in a case where the number of amplified loci is simply increased, the quantitative determination of the number of chromosomes cannot be accurately performed from a small amount of DNA of a single cell, a small number of cells, or the like.

From the viewpoint of the above-described circumstances, an object of the present invention is to provide a chromosome number determination method of chromosomes of interest in which it is possible to accurately perform quantitative determination of the number of chromosomes from a small amount of DNA of a single cell, a small number of cells, or the like.

The present inventors have conducted extensive studies to solve the above-described problems. As a result, they have found that, in a chromosome number determination method of chromosomes of interest which includes a step of performing multiplex PCR for simultaneously amplifying a plurality of loci on the chromosomes using genomic DNA extracted from a single cell or a small number of cells as templates, in cases where the number of loci on the chromosomes of interest is greater than or equal to 80 per chromosome and a method for designing primer sets used in the multiplex PCR is a method for designing primer sets in which a local alignment score is obtained by performing pairwise local alignment on a base sequence of a primer candidate under a condition that a partial sequence to be subjected to comparison includes the 3' terminal of the base sequence of the primer, first stage selection is performed while evaluating formability of a primer dimer based on the obtained local alignment score, a global alignment score is obtained by performing pairwise global alignment on a base sequence which has a sequence length of three bases and includes the 3' terminal of the base sequence of the primer candidate, second stage selection is performed while evaluating formability of the primer dimer based on the obtained global alignment score, and primers selected in both of the first stage and the second stage are employed, it is possible to accurately perform quantitative determination of the number of chromosomes from a small amount of DNA of a single cell, a small number of cells, or the like, and have completed the present invention.

That is, the present invention is as defined in claim 1.

Embodiments of the invention are defined in the dependent claims.

According to the present invention, it is possible to provide a chromosome number determination method of chromosomes of interest in which it is possible to accurately perform quantitative determination of the number of chromosomes which are objects of the quantitative determination of the number of chromosomes from a small amount of DNA of a single cell, a small number of cells, or the like.

In addition, according to the chromosome number determination method of the present invention, the method is not performed through whole genome amplification (WGA), and therefore, it is possible to eliminate bias caused by WGA in the related art.

## BRIEF DESCRIPTION OF THE DRAWINGS

Fig. 1 is a block diagram representing a method for designing primer sets in the present invention.

Fig. 2 is a diagram showing local alignment, a local alignment score, global alignment, and a global alignment score of a base sequence of SEQ ID No: 1 and a base sequence of SEQ ID No: 2.

Fig. 3 is a diagram showing local alignment, a local alignment score, global alignment, and a global alignment score of a base sequence of SEQ ID No: 21 and a base sequence of SEQ ID No: 22.

Fig. 4 is a diagram showing local alignment, a local alignment score, global alignment, and a global alignment score of a base sequence of SEQ ID No: 41 and a base sequence of SEQ ID No: 42.

Fig. 5 is a diagram showing local alignment, a local alignment score, global alignment, and a global alignment score of a base sequence of SEQ ID No: 61 and a base sequence of SEQ ID No: 62.

Fig. 6 is a diagram showing local alignment, a local alignment score, global alignment, and a global alignment score of a base sequence of SEQ ID No: 81 and a base sequence of SEQ ID No: 82.

Fig. 7 is a graph showing a relationship between the (total) number of loci and a coefficient of variation which are derived from results of an example and comparative examples. Plots and an approximate curve which represent data obtained from the example and the comparative examples are shown in the graph.

## DESCRIPTION OF THE PREFERRED EMBODIMENTS

Hereinafter, a chromosome number determination method of chromosomes of interest of the present invention will be described in detail.

In the present specification, the range represented by "to" means a range including both ends denoted before and after "to".

### [Step of Performing Multiplex PCR]

The step of performing multiplex PCR includes a step of performing multiplex PCR for simultaneously amplifying a plurality of loci on chromosomes, in which loci to be amplified exist, using genomic DNA extracted from a single cell or a small number of cells as templates.

#### <Genomic DNA Extracted from Single Cell or Small Number of Cells>

Genomic DNA extracted from a single cell or a small number of cells will be described below.

The "single cell" refers to one cell and a "small number of cells" refers to a number of cells of less than 10.

The genomic DNA refers to DNA extracted from a cell. Although the genomic DNA may be concentrated or diluted, a whole genome amplification product which is obtained by amplifying genomic DNA through whole genome amplification and a specific region amplification product obtained by amplifying a specific region of genomic DNA are not included in the genomic DNA.

<<Genomic DNA Extracted from Single Cell>>

A genomic DNA extracted from a single cell can be prepared, for example, by isolating a single cell from a population of cells and extracting the genomic DNA from the isolated single cell.

The method for isolating a single cell from a population of cells is not particularly limited, and a well-known method in the related art can be used. A method for isolating a single cell from a maternal blood sample will be described as an example. Even for samples other than the maternal blood sample, a method described below can be appropriately modified and used.

(Maternal Blood Sample)

The maternal blood sample is not particularly limited as long as the sample is a blood sample collected from a maternal body (pregnant woman), and maternal peripheral blood is preferable. Maternal body-derived nucleated red blood cells and fetus-derived nucleated red blood cells are included in the maternal peripheral blood in addition to white blood cells such as maternal body-derived eosinophils, neutrophils, basophils, mononuclear cells, and lymphocytes, and mature red blood cells having no nucleus. It has been known that fetus-derived nucleated red blood cells exist in maternal blood from about 6 weeks after pregnancy. For this reason, in the present invention, it is preferable to test peripheral blood of a pregnant woman after about 6 weeks of pregnancy.

(Fetal Nucleated Red Blood Cell)

The single cell is not particularly limited as long as the single cell is derived from a fetus, but a fetus-derived nucleated red blood cell is preferable. The fetus-derived nucleated red blood cell is a red blood cell precursor existing in maternal blood. During pregnancy of a mother, a red blood cell of a fetus may be nucleated. Since there is a chromosome in this red blood cell, a fetus-derived chromosome and a fetal gene become available using less invasive means. It has been known that this fetus-derived nucleated red blood cell exists at a rate of one in $10^6$ cells in the maternal blood, and the existence probability of the fetus-derived nucleated red blood cell in peripheral blood in a pregnant woman is extremely low.

(Concentration of Fetal Nucleated Red Blood Cell)

Fetus-derived nucleated red blood cells can be concentrated through density gradient centrifugation as a preferred embodiment in a case of isolating single cells.

The density of blood cells in a maternal body including fetus-derived nucleated red blood cells is disclosed in WO2012/023298A. According to the disclosure, the assumed density of the fetus-derived nucleated red blood cells is about 1.065 to 1.095 g/mL. On the other hand, the density of blood cells of the maternal blood is about 1.070 to 1.120 g/mL in a case of red blood cells, about 1.090 to 1.110 g/mL in a case of eosinophils, about 1.075 to 1.100 g/mL in a case of neutrophils, about 1.070 to 1.080 g/mL in a case of basophils, about 1.060 to 1.080 g/mL in a case of lymphocytes, and about 1.060 to 1.070 g/mL in a case of mononuclear cells.

In a case where fetus-derived nucleated red blood cells are concentrated through density gradient centrifugation, it is possible to use media such as Percoll (manufactured by GE Healthcare Bioscience) that is a silicic acid colloidal particle dispersion which is coated with polyvinylpyrrolidone and has a diameter of 15 to 30 nm, Ficoll-Paque (manufactured by GE Healthcare Bioscience) which is a neutral hydrophilic polymer which is rich in side chains and formed of sucrose, and/or Histopaque (manufactured by Sigma-Aldrich Co. LLC.) which is a solution using polysucrose and sodium diatrizoate, as a first medium and a second medium.

In the present invention, it is preferable to use Percoll and/or Histopaque. A product with a density of 1.130 g/cm$^3$ (specific gravity of 1.130) is commercially available as Percoll, and it is possible to prepare a medium with a target density (specific gravity) by diluting the product. In addition, a medium with a density of 1.077 g/cm$^3$ (specific gravity of 1.077) and a medium with a density of 1.119 g/cm$^3$ (specific gravity of 1.119) are commercially available as Histopaque, and it is possible to prepare a medium with a target density (specific gravity) by mixing these media with each other. By using Percoll and Histopaque, it is possible to prepare a first medium and a second medium.

The density of media to be stacked is set in order to separate fetus-derived nucleated red blood cells having a density of about 1.065 to 1.095 g/mL from other blood cell components in a maternal body. The central density of fetus-derived nucleated red blood cells is about 1.080 g/mL. Therefore, in a case where two media (first medium and second medium) having different densities interposing the central density are prepared and are made to be adjacent to and overlap each other, it is possible to collect fractions having the desired fetus-derived nucleated red blood cells on an interface between the media. It is preferable that the density of the first medium is set to be 1.080 g/mL to 1.100 g/mL and the density of the second medium is set to be 1.060 g/mL to 1.080 g/mL. It is more preferable that the density of the first medium is set to be 1.080 g/mL to 1.090 g/mL and the density of the second medium is set to be 1.065 g/mL to 1.080 g/mL. As a specific embodiment, it is preferable to separate plasma components, eosinophils, and mononuclear cells from the desired fractions to be collected, by setting the density of the first medium to 1.085 g/mL and the density of the second medium to 1.075 g/mL. In addition, by setting the densities of the media, it is also possible to partially separate red blood cells, neutrophils, and lymphocytes therefrom. In the present invention, the type of the first medium and the type of the second medium may be the same as or different from each other. However, the types of the media are preferably the same as each other.

(Sorting and Isolating of Nucleated Red Blood Cell Candidate)

Examples of a method of isolating a single cell include a method for peeling cells one by one from a transparent substrate with a micromanipulator, and sorting performed through immunological dyeing and fluorescence activated cell sorting (FACS).

Hereinafter, the method for peeling a single cell from a transparent substrate with a micromanipulator will be described in detail.

In order to obtain a nucleated red blood cell candidate from maternal blood, it is possible to prepare a substrate (blood cell specimen) coated with blood cells by coating the top of the substrate with blood and drying the blood. A transparent medium is preferably used as this substrate and slide glass is more preferably used as this substrate.

It is possible to sort out a fetus-derived nucleated red blood cell candidate based on the information on the shape of blood cells obtained from the blood cell specimen. As a preferred embodiment, it is possible to sort out a fetus-derived nucleated red blood cell candidate using a ratio of the area of a nuclear region to the area of cytoplasm of a cell, the degree of circularity of a nucleus, and/or the area of a nuclear region, and the like. Particularly, it is preferable to sort out a cell in which the ratio of the area of a nuclear region to the area of cytoplasm or the degree of circularity of a nucleus satisfies the conditions, as a fetus-derived nucleated red blood cell candidate.

In the present invention, it is preferable to sort out cells in which the ratio "N / C" of the area of a nuclear region to the area of cytoplasm satisfies Formula (1).

$$0.25 < N / C < 1.0 \cdots (1)$$

However, in Formula (1), "N" represents the area of a nuclear region of a cell on which image analysis is to be performed and "C" represents the area of cytoplasm of a cell on which image analysis is to be performed.

In addition, in the present invention, it is preferable to sort out cells in which the ratio "$N / L^2$" of the area of the nuclear region to the square of the length of the major axis of a nucleus satisfies Formula (2).

$$0.65 < N /L^2 < 0.785 \cdots (2)$$

However, in Formula (2), "N" represents the area of a nuclear region of a cell on which image analysis is to be performed and "L" represents the length of a major axis of a nucleus of a cell on which image analysis is to be performed, that is, the length of a major axis of an ellipse circumscribing a cell nucleus which has a complicated shape.

A system of sorting out a fetus-derived nucleated red blood cell candidate using information on the shape of cells is equipped with an optical microscope, a digital camera, a stage for slide glass, an optical transfer system, an image processing PC, a control PC, and a display. The optical transfer system includes an objective lens and a CCD camera. The image processing PC includes a processing system of performing data analysis and storing of data. The control PC includes a control system of controlling the position of a stage for slide glass or controlling the entire processing.

A protein existing in a red blood cell in the blood of all vertebrates including human beings is hemoglobin. The presence or absence of hemoglobin in a nucleated red blood cell is different from the presence or absence of hemoglobin in a white blood cell which is a type of nucleated cell in blood. Hemoglobin in a case of being bonded to oxygen is oxygenated hemoglobin exhibiting clear red color, and hemoglobin in a case of not being bonded to oxygen is reduced hemoglobin exhibiting dark red color. Hemoglobin having different oxygen bonding amounts flows in the arteries and the veins. Hemoglobin has absorption at 380 nm to 650 nm. Therefore, it is possible to detect hemoglobin using information of at least one monochromatic light beam caused by the difference in the absorbance of this wavelength range. It is preferable to use monochromatic light in order to check the presence or absence of hemoglobin. It is possible to select light with a single wavelength in a wavelength range of 400 nm to 500 nm or monochromatic light in a wavelength range of 525 nm to 580 nm, in which the absorbance of hemoglobin is large. The absorption coefficients of these wavelength ranges show high values due to the existence of hemoglobin. Therefore, the ratio of each absorption coefficient of these wavelength ranges to the absorption coefficient of cytoplasm of a white blood cell becomes greater than or equal to 1.

It is possible to identify a cell in which a cell nucleus having a nearly circular shape exists and which has hemoglobin, as a nucleated red blood cell candidate. Furthermore, in fetus-derived nucleated red blood cells and adult-derived nucleated red blood cells, hemoglobin of a fetus is hemoglobin F (HbF) and hemoglobin of an adult is hemoglobin A (HbA). Therefore, it is possible to sort out fetus-derived nucleated red blood cells using the difference in spectral characteristics caused by different oxygen bonding abilities.

In a case of measuring the absorption coefficient of cytoplasm, it is possible to use a micro spectrophotometer. The micro spectrophotometer is a photometer in which the same principle as that of a usual spectrophotometer is used for an optical system of a microscope, and it is possible to use a commercially available device.

In some cases, it is impossible to define whether the isolated nucleated red blood cell is derived from a fetus or from a maternal body (pregnant woman) depending on only the information on the shape and/or the absorbance of the cell. However, in the present invention, it is possible to discriminate the origin of the isolated nucleated red blood cell through polymorphism analysis using SNP and/or short tandem repeat (STR: short tandem repeat sequence) or the like, and through DNA analysis such as checking the presence of a Y chromosome.

(Extraction of Genomic DNA)

Extraction of genomic DNA from a single cell can be performed through a well-known method in the related art. It is preferable to use a commercially available DNA extraction kit. Examples of a commercially available DNA extraction kit that can be used for genomic DNA extraction from a single cell include Single Cell WGA Kit (manufactured by New England Biolabs). In a case where the commercially available DNA extraction kit is used, DNA extraction may be carried out according to the protocol attached to the kit, but the protocol may be appropriately modified and used.

<<Genomic DNA Extracted from Small Number of Cells>>

Genomic DNA extracted from a small number of cells can be prepared, for example, by separating a small number of cells from a population of cells, extracting genomic DNA from the small number of isolated cells, by isolating single cells from a population of cells, mixing the isolated single cells with each other, and extracting genomic DNA from a small number of the mixed cells, by isolating a single cell from a population of cells, extracting genomic DNA from the isolated single cell, and mixing the extracted genomic DNA's with each other, or by a combination of two or more of these methods.

<Multiplex PCR>

Multiplex PCR is PCR for simultaneously amplifying a plurality of loci on chromosomes using a plurality of primer sets.

<<Thermal Cycle>>

Multiplex PCR includes a plurality of thermal cycles including thermal denaturation, annealing, and elongation. The multiplex PCR may further include initial thermal denaturation and/or final elongation as desired.

(Thermal Denaturation)

The conditions of thermal denaturation such as the temperature and the time are not particularly limited as long as it is possible to dissociate two chains of genomic DNA to make a chain.

As examples of suitable conditions for thermal denaturation, the temperature is set to 90°C to 95°C and preferably to 94°C ± 2°C and the time is set to 10 seconds to 60 seconds and preferably 30 seconds ± 5 seconds.

The temperature and time of thermal denaturation may be appropriately changed depending on the amount of genomic DNA of templates.

(Annealing)

The conditions of annealing such as the temperature and the time are not particularly limited as long as it is possible to bond a primer to genomic DNA which has been disassociated to become a chain.

As examples of suitable conditions for annealing, the temperature is set to 50°C to 65°C and preferably to 60°C ± 2°C and the time is set to 10 seconds to 90 seconds and preferably 60 ± 10 seconds.

The temperature and time of annealing may be appropriately changed depending on the GC content (referring to a total mole percentage of guanine (abbreviation = G) and cytosine (abbreviation = C) in all nucleic acid bases) of a primer, a Tm value (which is a temperature at which 50% of double-stranded DNA is dissociated and becomes single-stranded DNA and in which Tm is derived from a melting temperature), and deviation of a sequence.

(Elongation)

The elongation condition is not particularly limited as long as it is a temperature and time at which the polynucleotide chain can be elongated from the 3' terminal of a primer by DNA polymerase.
As examples of suitable conditions for elongation, the temperature is set to 72°C ± 2°C and the time is set to 10 seconds to 60 seconds and preferably 30 ± 5 seconds.
The temperature and time for elongation may be appropriately changed depending on the type of DNA polymerase and/or the size of PCR amplification product.

(Initial Thermal Denaturation)

Initial thermal denaturation may be performed before the first cycle of a thermal cycle is started.
The conditions of initial thermal denaturation may be the same as or different from the conditions of the thermal denaturation. In the case where the conditions of initial thermal denaturation are different from the conditions of the thermal denaturation, it is preferable to set the temperature at the same temperature as in the case of the thermal denaturation and set the time to be longer than that of the thermal denaturation.
By performing the initial thermal denaturation, it is possible to more reliably dissociate two chains of genomic DNA in the first cycle of the thermal cycle.

(Final Elongation)

Final elongation may be performed after the last cycle of the thermal cycle is completed.
The conditions of final elongation may be the same as or different from the conditions of the elongation. In the case where the conditions of final elongation are different from the conditions of the elongation, it is preferable to set the temperature at the same temperature as in the case of the thermal denaturation and set the time to be longer than that of the elongation.
By performing the final elongation, it is possible to more reliably elongate a polynucleotide chain.

(Number of Cycles)

The number of cycles is not particularly limited as long as it is plural, but is preferably 20 cycles to 40 cycles and more preferably 35 ± 5 cycles.
The number of cycles may be appropriately changed depending on the amount of genomic DNA which becomes a template of multiplex PCR, the number of primer sets used for the multiplex PCR, and/or the amount of reaction solution of multiplex PCR.
Although the amplification product obtained through PCR theoretically increases twice per cycle, in reality, it reaches a plateau in a certain cycle, and there is a possibility that amplification products more than that may not be desired or a nonspecific amplification product may increase. Therefore, it cannot be said that it is desirable to increase the number of cycles unconditionally.

(Primer Set)

A primer set designed according to "Method for Designing Primer Sets" to be described below can be used as a primer set used in multiplex PCR, as done in the present invention. Since the primer set is designed not to form a primer dimer, it is possible to suppress the increase in the nonspecific amplification product and to improve the sensitivity of the multiplex PCR itself.
The number of primer sets is set corresponding to the number of loci to be amplified. An identical locus may be amplified by two or more pairs of primer sets, or two or more loci may be amplified by a pair of primer sets. In general, it is preferable that the locus corresponds one to one to the primer set.

<<Plurality of Loci on Chromosomes>>

(Chromosomes)

Chromosomes include one or more selected from the group consisting of an autosome from chromosome 1 to chromosome 22 of a human and a sex chromosome of X and Y chromosomes.

The chromosomes are not particularly limited as long as these include chromosomes (in the present invention, in particular, there are some cases in which these may be referred to as "chromosomes of interest") to be subjected to quantitative determination of the number of chromosomes.

The chromosomes may include a chromosome that provides a reference value for quantitative determination of chromosomes and/or a chromosome that is only interested in the presence or absence of loci, in addition to the chromosomes to be subjected to quantitative determination of the number of chromosomes. That is, even in a case of chromosomes in which loci to be amplified through multiplex PCR exist, the chromosome that provides a reference value for quantitative determination of chromosomes and/or a chromosome that is only interested in the presence or absence of loci are excluded from the chromosomes (chromosomes of interest) to be subjected to quantitative determination of the number of chromosomes.

The chromosomes of interest particularly preferably contain at least one selected from the group consisting of chromosome 13, chromosome 18 and chromosome 21. These chromosomes are more likely to generate trisomy or monosomy compared to other autosomes.

Examples of the chromosome that provides a reference value for quantitative determination of chromosomes include an autosome and/or an X chromosome other than the chromosomes which are likely to generate trisomy or monosomy. Among them, the X chromosome is a preferred chromosome because it exists regardless of the gender of men and women.

An example of the chromosome that is only interested in the presence or absence of loci includes a Y chromosome. This is because the presence of the Y chromosome strongly suggests male among the genders of men and women. In a case of quantitatively determining the number of chromosomes of a fetus, it is preferable that the chromosomes include a Y chromosome in order to discriminate cells derived from a mother (maternal boy) from cells derived from a fetus. This is because the presence of the Y chromosome suggests a denial of the origin of cells derived from a mother (maternal body).

(Plurality of Loci)

A plurality of loci are loci to be amplified through multiplex PCR out of loci on chromosomes.

Since the chromosomes may include chromosome that provides a reference value for quantitative determination of chromosomes and/or a chromosome that is only interested in the presence or absence of loci in addition to the chromosomes (chromosomes of interest) to be subjected to quantitative determination of the number of chromosomes, the plurality of loci are not limited to those existing on the chromosomes to be subjected to quantitative determination of the number of chromosomes and may include loci existing on the chromosome that provides a reference value for quantitative determination of chromosomes and/or loci existing on the chromosome that is only interested in the presence or absence of loci.

The loci may exist in either a gene region or a non-gene region.

The gene region includes: a coding region in which a gene encoding proteins, a ribosomal ribonucleic acid (RNA) gene, a transfer RNA gene, and the like exist; and a non-coding region in which an intron dividing a gene, a transcription regulatory region, a 5' leader sequence, a 3' trailer sequence, and the like exist.

The non-gene region includes: a non-repetitive sequence such as a pseudogene, a spacer, a response element, and a replication origin; and a repetitive sequence such as a tandem repetitive sequence and an interspersed repetitive sequence.

Loci may be, for example, loci such as single nucleotide polymorphism (SNP), single nucleotide variant (SNV), short tandem repeat polymorphism (STRP), mutation, and insertion and/or deletion (indel).

(Number of Loci)

The number of loci on the chromosomes (chromosomes of interest) to be subjected to quantitative determination of the number of chromosomes is not particularly limited as long as it is greater than or equal to 80 and the chromosomes are chromosomes of interest, but is preferably 80 to 1,000, more preferably 100 to 1,000, still more preferably 100 to 500, and still more preferably 100 to 200. In particular, the number of loci per chromosome is preferably 150 to 200.

In a case where the number of loci per chromosome of interest is within this ranges, the coefficient of variation of the coverage becomes sufficiently small, and therefore, the accuracy of the quantitative determination of the number of chromosomes can be improved.

[Step of Quantitatively Determining Number of Chromosomes]

In the present invention, the quantitative determination of the number of chromosomes can be carried out through a well-known method in the related art, but is preferably carried out, for example, through a method to be described below using a next generation sequencer.

It is desirable to particularly use Miseq (manufactured by Illumina, Inc.) as the next generation sequencer. In a case of sequencing a plurality of multiplex PCR amplification products using the next generation sequencer "Miseq", it is necessary to add P5 and P7 sequences, which are used for hybridizing to a sample identification sequence (index sequence) formed of 6 to 8 bases, and an oligonucleotide sequence on the top of a Miseq flow cell, to each of the multiplex PCR amplification products. By adding these sequences thereto, it is possible to measure up to 96 types of multiplex PCR amplification products at a time.

It is possible to use an adapter ligation method or a PCR method as the method for adding an index sequence and P5 and P7 sequences to both terminals of the multiplex PCR amplification products.

As the method for analyzing sequence data obtained using Miseq to quantitatively determine the number of chromosomes, it is preferable to map the sequence data in a well-known human genome sequence using Burrows-Wheeler Aligner (BWA: Li, H., et al., "Fast and accurate short read alignment with Burrows-Wheeler transform", Bioinformatics, 2009, Vol. 25, No. 14, PP. 1754-1760; and Li, H., et al., "Fast and accurate long-read alignment with Burrows-Wheeler transform", Bioinformatics, 2010, Vol. 26, No. 5, PP. 589-595). As means for analyzing a genetic abnormality, it is preferable to quantitatively determine the number of chromosomes using SAMtools (Li, Heng, et al., "The Sequence Alignment / Map format and SAMtools", Bioinformatics, 2009, Vol. 25, No. 16, PP. 2078-2079; SAM is derived from "Sequence Alignment / Map") and/or BEDtools (Quinlan, A. R., et al., "BEDtools: a flexible suite of utilities for comparing genomic features", Bioinformatics, 2010, Vol. 26, No. 6, PP. 841-842).

For example, regarding DNA fragments in which fetal nucleated red blood cells are identified and which are obtained by performing PCR amplification of a target locus, the amplification amount (the coverage, the sequence depth, and the number of times of sequence reading) of amplification product having a sequence of a region of 140 bp to 180 bp which has been previously determined can be obtained using a sequencer.

Regarding a cell which has been identified as a mother-derived nucleated red blood cell, the amplification amount (number of times of sequence reading) of amplification product having a sequence of a region of 140 bp to 180 bp which has been previously determined is obtained as a standard (reference) using the sequencer. In a case where fetuses are in normal states, it is expected that the ratio of the amplification amount (number of times of sequence reading) of mother-derived amplification product to the amplification amount (number of times of sequence reading) of fetus-derived amplification product becomes almost 1:1. In a case where fetuses have a disease which is trisomy derived from an amplified chromosome, it is expected that the ratio thereof becomes almost 1:1.5 (or 2:3).

In the present invention, the proportions of the amount (number of times of sequence reading) of fetus-derived PCR amplification products to the amount (number of times of sequence reading) of mother-derived PCR amplification products which have been collected from a plurality of pregnant maternal bodies in a case where the mothers are pregnant with normal fetuses are obtained plural times, and the distribution thereof is obtained. In addition, the proportions of the amount (number of times of sequence reading) of fetus-derived amplification products to the amount (number of times of sequence reading) of mother-derived amplification products in a case where the mothers are pregnant with fetuses with trisomy are obtained, and the distribution thereof is obtained. It is also possible to set a cutoff value in a region where these two distributions do not overlap. After comparing the cutoff value which has previously been determined with a result in which the proportion of the amplification products is obtained, it is also possible to interpret inspection results that the fetuses are normal in a case where the proportion thereof is less than or equal to the cutoff value, and the fetuses have trisomy in a case where the proportion thereof is greater than or equal to the cutoff value.

[Method for Designing Primer Sets]

Hereinafter, the method for designing primer sets which is one of the characteristic features will be described in detail.

The method for designing primer includes the following steps:

(a) a target locus selection step of selecting a target locus for designing primer sets used in the multiplex PCR from the plurality of loci;

(b) a primer candidate base sequence generation step of generating at least one base sequence of a primer candidate for amplifying the target locus regarding each of a forward-side primer and a reverse-side primer based on a base sequence in a vicinity region of the target locus on the chromosomes;

(c) a local alignment step of obtaining a local alignment score by performing pairwise local alignment on the base sequence of the primer candidate for amplifying the target locus under a condition that a partial sequence to be subjected to comparison includes the 3' terminal of the base sequence of the primer candidate for amplifying the target locus;

(d) a first stage selection step of performing first stage selection of the base sequence of the primer candidate for amplifying the target locus based on the local alignment score obtained in the local alignment step;

(e) a global alignment step of obtaining a global alignment score by performing pairwise global alignment on a base sequence which has a sequence length of three bases and includes the 3' terminal of the base sequence of the primer candidate for amplifying the target locus;

(f) a second stage selection step of performing second stage selection of the base sequence of the primer candidate for amplifying the target locus based on the global alignment score obtained in the global alignment step; and

(g) a primer employment step of employing the base sequence of the primer candidate which has been selected in both of the first stage selection step and the second stage selection step as the base sequence of the primer for amplifying the target locus.

Both steps of (c) Local Alignment Step and (d) First Stage Selection Step are performed before or after both steps of (e) Global Alignment Step and (f) Second Stage Selection Step, or performed in parallel with both steps of (e) Global Alignment Step and (f) Second Stage Selection Step.

Each step of the method for designing primer sets in the present invention will be described in detail. This method will be sometimes referred to as the "first embodiment" of the method for designing primer sets in the present invention, while a specific embodiment which is not part of the invention will be referred to as the "second embodiment" of the method for designing primer sets.

The specific second embodiment will be described in detail further below.

(a) Target Locus Selection Step

(a) Target Locus Selection Step is shown in the block diagram of Fig. 1 as "(n-th) TARGET LOCUS SELECTION STEP".

The target locus selection step is a step of selecting a locus (target locus) primer for designing sets used in the multiplex PCR from the plurality of loci.

The number of a plurality of loci to be amplified through multiplex PCR is N (where N is an integer satisfying N $\geq$ 2) and the number of target loci that can be selected is n (n is an integer satisfying 1 $\leq$ n $\leq$ N).

In a case where two or more loci are selected, successive primer sets may be designed for each locus, primer sets may be designed in parallel for each locus, or primer sets may be designed at the same time for each locus.

(b) Primer Candidate Base Sequence Generation Step

(b) Primer Candidate Base Sequence Generation Step is shown in the block diagram of Fig. 1 as "(n-th) PRIMER CANDIDATE BASE SEQUENCE GENERATION STEP".

The primer candidate base sequence generation step is a step of generating at least one base sequence of a primer candidate for amplifying the target locus regarding each of a forward-side primer and a reverse-side primer based on a base sequence in a vicinity region of the target locus on the chromosomes.

A base sequence of a primer candidate is generated based on the base sequence of the above-described vicinity region, but may have a portion not complementary to the base sequence of the above-described vicinity region at a 5' terminal side. In some cases, such a portion not complementary to the 5' terminal side of the primer may be used to add a specific base sequence to an amplification product obtained through multiplex PCR.

The vicinity region of a target locus is a region excluding the target locus in a region including the target locus on a chromosome.

The length of a vicinity region is not particularly limited, but is preferably less than or equal to a length that can be expanded through PCR and more preferably less than or equal to the upper limit of a fragment length of DNA for which amplification is desired. A length facilitating application of concentration selection and/or sequence reading is particularly preferable. The length of a vicinity region may be appropriately changed in accordance with the type of enzyme (DNA polymerase) used for PCR. The specific length of a vicinity region is preferably about 20 to 500 bases, more preferably about 20 to 300 bases, still more preferably about 20 to 200 bases, and particularly preferably about 50 to 200 bases.

In addition, in a case of generating a base sequence of a primer candidate, points, such as the length of a complementary portion of a primer, the total length of a primer, the GC content (referring to a total mole percentage of guanine (abbreviation = G) and cytosine (abbreviation = C) in all nucleic acid bases), a Tm value (which is a temperature at which 50% of double-stranded DNA is dissociated and becomes single-stranded DNA and in which Tm is derived from a melting temperature), and deviation of a sequence, to be taken into consideration in a general method for designing a primer are the same.

The complementary portion of a primer is a portion at which the primer hybridizes to single-stranded DNA of a template during annealing. A base sequence of a complementary portion of a primer is generated based on a base sequence of a vicinity region of a target locus. In the present invention, a non-complementary portion may be linked to the 5' terminal of the complementary portion of the primer. The non-complementary portion is a portion at which the primer is not intended to hybridize to single-stranded DNA of template DNA. As the base sequence of the non-complementary portion of the primer, there is a tail sequence used for adding a sequence for sequencing to an amplification product, which is obtained through multiplex PCR, by performing PCR (second PCR) using the amplification product as a template.

The length of a complementary portion of a primer (the number of nucleotides) is not particularly limited, but is preferably 10 mer to 30 mer, more preferably 15 mer to 30 mer, and still more preferably 15 mer to 25 mer. In a case where the length of a complementary portion of a primer is within this range, it is easy to design a primer excellent in specificity and amplification efficiency.

The GC content is not particularly limited, but is preferably 40 mol% to 60 mol% and more preferably 45 mol% to 55 mol%. In a case where the GC content is within this range, a problem such as a decrease in the specificity and the amplification efficiency due to a high-order structure is less likely to occur.

The Tm value is not particularly limited, but is preferably within a range of 50°C to 65°C and more preferably within a range of 55°C to 65°C.

The Tm value can be calculated using software such as OLIGO Primer Analysis Software (manufactured by Molecular Biology Insights) or Primer 3 (http://www-genome.wi.mit.edu/ftp/distribution/software/).

In addition, the Tm value can also be obtained through calculation using the following formula from the number of A's, T's, G's, and C's (which are respectively set as nA, nT, nG, and nC) in a base sequence of a primer.

$$\text{Tm value } (^\circ\text{C}) = 2(\text{nA} + \text{nT}) + 4(\text{nC} + \text{nG})$$

The method for calculating the Tm value is not limited thereto and can be calculated through various well-known methods in the related art.

The base sequence of a primer candidate is preferably set as a sequence in which there is no deviation of bases as a whole. For example, it is desirable to avoid a GC-rich sequence and a partial AT-rich sequence.

In addition, it is also desirable to avoid continuation of T and/or C (polypyrimidine) and continuation of A and/or G (polypurine).

Furthermore, it is preferable that a 3' terminal base sequence avoids a GC-rich sequence or an AT-rich sequence. G or C is preferable for a 3' terminal base, but is not limited thereto.

(Specificity-Checking Step)

As desired, a specificity-checking step may be performed.

The specificity-checking step is a step of evaluating specificity of a base sequence of a primer candidate may be performed based on sequence complementarity with respect to genomic DNA of a base sequence of each primer candidate which has been generated in (b) Primer Candidate Base Sequence Generation Step.

In the specificity check, in a case where local alignment of a base sequence of genomic DNA and a base sequence of a primer candidate is performed and a local alignment score is less than a predetermined value, it is possible to evaluate that the complementarity of the base sequence of the primer candidate with respect to genomic DNA is low and the specificity of the base sequence of the primer candidate with respect to genomic DNA is high. Here, it is desirable to perform local alignment on also a complementary chain of genomic DNA. This is because genomic DNA is double-stranded whereas the primer is single-stranded DNA. In addition, a base sequence complementary to the base sequence of the primer candidate may be used instead of the base sequence of the primer candidate. The complementarity can be considered as homology with respect to a complementary chain.

In addition, homology search may be performed on genomic DNA base sequence database using the base sequence of the primer candidate as a query sequence. Examples of a homology search tool include Basic Local Alignment Search Tool (BLAST) (Altschul, S. A., et al., "Basic Local Alignment Search Tool", Journal of Molecular Biology, 1990, October, Vol. 215, pp. 403-410) and FASTA (Pearson, W. R., et al., "Improved tools for biological sequence comparison", Proceedings of the National Academy of Sciences of the United States of America, National Academy of Sciences, 1988, April, Vol. 85, pp. 2444-2448). It is possible to obtain local alignment as a result of performing the homology search.

Scores given to each of a complementary base (match), a non-complementary base (mismatch) and a gap (insertion and/or deletion (indel)) (in some cases, referred to as a "scoring system" in the present specification), and a threshold value of a local alignment score are not particularly limited, and can be appropriately set depending on the length of a base sequence of a primer candidate and/or the PCR conditions. In a case of using a homology search tool, a default value of the homology search tool may be used. For example, as the scoring system, it is considered that complementary base (match) = +1, non-complementary base (mismatch) = -1, and gap (insertion and/or deletion (indel)) = -3 are employed and the threshold value is set to be +15. In some cases, the score for the gap is referred to as a gap penalty.

In a case where a base sequence of a primer candidate has complementarity to a base sequence at an unexpected position on genomic DNA but has low specificity thereto, in some cases, an artifact is amplified instead of amplifying a target locus in a case where PCR is performed using a primer of the base sequence of a primer candidate. Therefore, the case where the base sequence of the primer candidate has complementarity to the base sequence at an unexpected position on genomic DNA but has low specificity thereto is excluded.

(c) Local Alignment Step

(c) Local Alignment Step is shown in the block diagram of Fig. 1 as "(n-th) LOCAL ALIGNMENT STEP".

The local alignment step is a step of obtaining a local alignment score by performing pairwise local alignment on the base sequence of the primer candidate for amplifying the target locus under a condition that a partial sequence to be subjected to comparison includes the 3' terminal of the base sequence of the primer candidate for amplifying the target locus.

A combination of pairs of base sequences to be subjected to local alignment may be a combination selected while allowing overlapping, or may be a combination selected without allowing overlapping. However, in a case where formability of a primer dimer between primers of an identical base sequence has not yet been evaluated, the combination selected while allowing overlapping is preferable.

The total number of combinations is "$_{m}H_{2} = {}_{m+1}C_{2} = (m+1)! / 2(m-1)!$" in a case where the selection is performed while allowing overlapping, and is "$_{m}C_{2} = m(m-1) / 2$" in a case where the selection is performed without allowing overlapping, in which the number of base sequences which have been generated in (b) Primer Candidate Base Sequence Generation Step is set to be m.

In a case where both steps of (e) Global Alignment Step and (f) Second Stage Selection Step to be described below are performed first, the present step and (d) First Stage Selection Step to be described below may be performed on primer candidates selected in (f) Second Stage Selection Step.

Local alignment is alignment which is performed on a partial sequence and in which it is possible to locally check a portion with high complementarity.

However, in the present invention, the local alignment is different from local alignment usually performed on a base sequence, and is designed such that partial sequences to be subjected to comparison include the 3' terminals of both base sequences by performing local alignment under the condition that the "partial sequences to be subjected to comparison include the 3' terminals of the base sequences". Furthermore, in the present invention, an embodiment is preferable in which partial sequences to be subjected to comparison include the 3' terminals of both base sequences by performing local alignment under the condition that the "partial sequences to be subjected to comparison include the 3' terminals of the base sequences", that is, the condition that "only alignments in which a partial sequence to be subjected to comparison begins at the 3' terminal of one sequence and ends at the 3' terminal of the other sequence".

Local alignment may be performed by inserting a gap. The gap means insertion and/or deletion (indel) of a base.

In addition, in the local alignment, a case where bases are complementary to each other between base sequence pairs is regarded as a match and a case where bases are not complementary to each other therebetween is regarded as a mismatch.

Local alignment is performed such that scores for each of the match, the mismatch, and the indel are given and the total score (local alignment score) becomes a maximum. The scores to be given to each of the match, the mismatch, and the indel may be appropriately set. For example, scores to be given to each of the match, the mismatch, and the indel may be set as shown in Table 1. "-" in Table 1 represents a gap (insertion and/or deletion (indel)).

[Table 1]

| | A | T | G | C | - |
|---|---|---|---|---|---|
| A | -1 | +1 | -1 | -1 | -3 |
| T | +1 | -1 | -1 | -1 | -3 |

(continued)

| | A | T | G | C | - |
|---|---|---|---|---|---|
| G | -1 | -1 | -1 | +1 | -3 |
| C | -1 | -1 | +1 | -1 | -3 |
| - | -3 | -3 | -3 | -3 | |
| "-": Gap | | | | | |

For example, it is considered that local alignment is performed on base sequences of SEQ ID No: 1 and SEQ ID No: 2 shown in Table 2. Here, the scores to be given to each of the match, the mismatch, and the gap are as shown in Table 1.

[Table 2]

| | Base sequence (5' → 3') |
|---|---|
| SEQ ID No: 1: | CGCTCTTCCGATCTCTGCTTCGATGCGGACCTTCTGG |
| SEQ ID No: 2: | CGCTCTTCCGATCTGACTCTCCCACATCCGGCTATGG |

From the base sequences of SEQ ID No: 1 and SEQ ID No: 2, a dot matrix shown in Table 3 is generated. Specifically, the base sequence of SEQ ID No: 1 is arranged from the left to the right in an orientation of 5' to 3' and the base sequence of SEQ ID No: 2 is arranged from the bottom to the top in an orientation of 5' to 3'. "·" is filled in a grid of which bases are complementary to each other, and a dot matrix shown in Table 3 is obtained.

[Table 3]

SEQ ID No: 1 →

SEQ ID No: 2: →

| | C | G | C | T | C | T | T | C | C | G | A | T | C | T | C | T | G | C | T | T | C | G | A | T | G | C | G | G | A | C | C | T | T | C | T | G | G |
|---|---|---|---|---|---|---|---|---|---|---|---|---|---|---|---|---|---|---|---|---|---|---|---|---|---|---|---|---|---|---|---|---|---|---|---|---|---|
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| T |  |  |  |  |  |  |  |  |  |  | ● |  |  |  |  |  |  |  |  |  |  |  | ● |  |  |  |  |  | ● |  |  |  |  |  |  |  |  |
| A |  |  |  | ● |  | ● | ● |  |  |  |  | ● |  | ● |  | ● |  |  | ● | ● |  |  |  | ● |  |  |  |  |  |  |  | ● | ● |  | ● |  |  |
| T |  |  |  |  |  |  |  |  |  |  | ● |  |  |  |  |  |  |  |  |  |  |  | ● |  |  |  |  |  | ● |  |  |  |  |  |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| T |  |  |  |  |  |  |  |  |  |  | ● |  |  |  |  |  |  |  |  |  |  |  | ● |  |  |  |  |  | ● |  |  |  |  |  |  |  |  |
| A |  |  |  | ● |  | ● | ● |  |  |  |  | ● |  | ● |  | ● |  |  | ● | ● |  |  |  | ● |  |  |  |  |  |  |  | ● | ● |  | ● |  |  |
| C |  |  |  |  |  |  |  |  |  |  |  |  |  |  |  |  |  |  |  |  |  |  |  |  |  |  |  |  |  |  |  |  |  |  |  |  |  |
| A |  |  |  | ● |  | ● | ● |  |  |  |  | ● |  | ● |  | ● |  |  | ● | ● |  |  |  | ● |  |  |  |  |  |  |  | ● | ● |  | ● |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| T |  |  |  |  |  |  |  |  |  |  | ● |  |  |  |  |  |  |  |  |  |  |  | ● |  |  |  |  |  | ● |  |  |  |  |  |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| T |  |  |  |  |  |  |  |  |  |  | ● |  |  |  |  |  |  |  |  |  |  |  | ● |  |  |  |  |  | ● |  |  |  |  |  |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| A |  |  |  | ● |  | ● | ● |  |  |  |  | ● |  | ● |  | ● |  |  | ● | ● |  |  |  | ● |  |  |  |  |  |  |  | ● | ● |  | ● |  |  |
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| T |  |  |  |  |  |  |  |  |  |  | ● |  |  |  |  |  |  |  |  |  |  |  | ● |  |  |  |  |  | ● |  |  |  |  |  |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| T |  |  |  |  |  |  |  |  |  |  | ● |  |  |  |  |  |  |  |  |  |  |  | ● |  |  |  |  |  | ● |  |  |  |  |  |  |  |  |
| A |  |  |  | ● |  | ● | ● |  |  |  |  | ● |  | ● |  | ● |  |  | ● | ● |  |  |  | ● |  |  |  |  |  |  |  | ● | ● |  | ● |  |  |
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| T |  |  |  |  |  |  |  |  |  |  | ● |  |  |  |  |  |  |  |  |  |  |  | ● |  |  |  |  |  | ● |  |  |  |  |  |  |  |  |
| T |  |  |  |  |  |  |  |  |  |  | ● |  |  |  |  |  |  |  |  |  |  |  | ● |  |  |  |  |  | ● |  |  |  |  |  |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| T |  |  |  |  |  |  |  |  |  |  | ● |  |  |  |  |  |  |  |  |  |  |  | ● |  |  |  |  |  | ● |  |  |  |  |  |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |
| G | ● |  | ● |  | ● |  |  | ● | ● |  |  |  | ● |  | ● |  |  | ● |  |  | ● |  |  |  |  | ● |  |  |  | ● | ● |  |  | ● |  |  |  |
| C |  | ● |  |  |  |  |  |  |  | ● |  |  |  |  |  |  | ● |  |  |  |  | ● |  |  | ● |  | ● | ● |  |  |  |  |  |  |  | ● | ● |

From the dot matrix shown in Table 3, alignment (pairwise alignment) of partial sequences shown in Table 4 is obtained (refer to a thick line portion of Table 3).

[Table 4]

<table>
<tr><th colspan="23">Base sequence</th></tr>
<tr><td>Partial sequence from SEQ ID No: 1:</td><td>5'·</td><td>C</td><td>T</td><td>T</td><td>C</td><td>G</td><td>A</td><td>T</td><td>G</td><td>C</td><td>G</td><td>G</td><td>A</td><td>C</td><td>C</td><td>T</td><td>T</td><td>C</td><td>T</td><td>G</td><td>G</td><td>3'</td></tr>
<tr><td></td><td></td><td>|</td><td>:</td><td>:</td><td>:</td><td>:</td><td>:</td><td>:</td><td>*</td><td>|</td><td>|</td><td>|</td><td>|</td><td>:</td><td>:</td><td>|</td><td>:</td><td>:</td><td>:</td><td>:</td><td>|</td><td></td></tr>
<tr><td>Partial sequence from SEQ ID No: 2:</td><td>3'·</td><td>G</td><td>G</td><td>T</td><td>A</td><td>T</td><td>C</td><td>G</td><td>-</td><td>G</td><td>C</td><td>C</td><td>T</td><td>A</td><td>C</td><td>A</td><td>C</td><td>C</td><td>C</td><td>T</td><td>C</td><td>·5'</td></tr>
<tr><td colspan="23">"|" = Match; ":" = Mismatch; "*" = Gap (indel)</td></tr>
</table>

Due to match (+1) × 7, mismatch (-1) × 12, and gap (-3) × 1 from Table 4, the local alignment score regarding the local alignment is "-8".

The alignment (pairwise alignment) can be obtained not only through the dot matrix method exemplified herein, but also through a dynamic programming method, a word method, or various other methods.

(d) First Stage Selection Step

(d) First Stage Selection Step is shown in the block diagram of Fig. 1 as "(n-th) STEP OF FIRST STAGE SELECTION".

The first stage selection step is a step of performing first stage selection of the base sequence of the primer candidate for amplifying the target locus based on the local alignment score obtained in (c) Local Alignment Step.

A threshold value (first threshold value) of the local alignment score is predetermined.

In a case where a local alignment score of a pair of two base sequences is less than the first threshold value, it is determined that the pair of these two base sequences has low primer dimer formability, and the following step is performed. In contrast, in a case where a local alignment score of a pair of two base sequences is greater than or equal to the first threshold value, it is determined that the pair of these two base sequences has high primer dimer formability, and the following step is not performed on the pair.

The first threshold value is not particularly limited and can be appropriately set. For example, the first threshold value may be set using a PCR condition such as the amount of genomic DNA which becomes a template for a polymerase chain reaction.

Here, in the example in which (c) Local Alignment Step is shown, a case where the first threshold value is set to "3" is considered.

In the above-described example, the local alignment score is "-8" and is less than "3" which is the first threshold value. Therefore, it is possible to determine that the pair of the base sequences of SEQ ID No: 1 and SEQ ID No: 2 has low primer dimer formability.

The present step is performed on all of the pairs for which scores are calculated in (c) Local Alignment Step.

(e) Global Alignment Step

(e) Global Alignment Step is shown in the block diagram of Fig. 1 as "(n-th) GLOBAL ALIGNMENT STEP".

The global alignment step is a step of obtaining a global alignment score by performing pairwise global alignment on a base sequence which has a sequence length of three bases and includes the 3' terminal of the base sequence of the primer candidate for amplifying the target locus.

A combination of pairs of base sequences to be subjected to global alignment may be a combination selected while allowing overlapping, or may be a combination selected without allowing overlapping. However, in a case where formability of a primer dimer between primers of an identical base sequence has not yet been evaluated, the combination selected while allowing overlapping is preferable.

The total number of combinations is "$_mH_2 = {}_{m+1}C_2 = (m+1)! / 2(m-1)!$" in a case where the selection is performed while allowing overlapping, and is "$_mC_2 = m(m-1) / 2$" in a case where the selection is performed without allowing overlapping, in which the number of base sequences which have been generated in (b) Primer Candidate Base Sequence Generation Step is set to be m.

In a case where both steps of (c) Local Alignment Step and (d) First Stage Selection Step which have been described above are performed first, the present step and (f) Second Stage Selection Step to be described below may be performed on primer candidates selected in (d) First Stage Selection Step.

Global alignment is an alignment which is performed on the entire sequence and in which it is possible to check complementarity of the entire sequence.

However, here, the "entire sequence" refers to the entirety of a base sequence which has a sequence length of three bases and includes the 3' terminal of a base sequence of a primer candidate.

Global alignment may be performed by inserting a gap. The gap means insertion and/or deletion (indel) of a base.

In addition, in the global alignment, a case where bases are complementary to each other between base sequence pairs is regarded as a match and a case where bases are not complementary to each other therebetween is regarded as a mismatch.

Global alignment is performed such that scores for each of the match, the mismatch, and the indel are given and the total score (global alignment score) becomes a maximum. The scores to be given to each of the match, the mismatch, and the indel may be set appropriately. For example, scores to be given to each of the match, the mismatch, and the indel may be set as shown in Table 1. "-" in Table 1 represents a gap (insertion and/or deletion (indel)).

For example, it is considered that global alignment is performed on three bases (refer to portions with capital letters and correspond to the "base sequence which has a sequence length of three bases and includes the 3' terminal") at the 3’ terminal of each base sequence of SEQ ID No: 1 and SEQ ID No: 2 shown in Table 5. Here, the scores to be given to each of the match, the mismatch, and the gap are as shown in Table 1.

[Table 5]

| | Base sequence (5’ → 3’) |
|---|---|
| SEQ ID No: 1: | cgctcttccgatctctgcttcgatgcggaccttcTGG |
| SEQ ID No: 2: | cgctcttccgatctgactctcccacatccggctaTGG |

Alignment (pairwise alignment) shown in Table 6 is obtained by performing global alignment on base sequences of the three bases (portion with capital letters) at the 3’ terminal of the base sequence of SEQ ID No: 1 and the three bases (portion with capital letters) at the 3’ terminal of SEQ ID No: 2 such that the score becomes a maximum.

[Table 6]

| | Base sequence | | | | |
|---|---|---|---|---|---|
| Three bases at 3’ terminal of SEQ ID No: 1: | 5’· | T | G | G | ·3’ |
| Three bases at 3’terminal of SEQ ID No: 2: | 3’· | G | G | T | ·5’ |
| ":" = Mismatch | | | | | |

Due to match (+1) × 0, mismatch (-1) × 3, and gap (-3) × 0 from Table 6, the global alignment score regarding the global alignment is "-3".

The alignment (pairwise alignment) can be obtained through the dot matrix method a dynamic programming method, a word method, or various other methods.

(f) Second Stage Selection Step

(f) Second Stage Selection Step is shown in the block diagram of Fig. 1 as "(n-th) STEP OF SECOND STAGE SELECTION".

The second stage selection step is a step of performing second stage selection of the base sequence of the primer candidate for amplifying the target locus based on the global alignment score obtained in (e) Global Alignment Step.

A threshold value (second threshold value) of the global alignment score is predetermined.

In a case where a global alignment score of a pair of two base sequences is less than the second threshold value, it is determined that the pair of these two base sequences has low primer dimer formability, and the following step is performed. In contrast, in a case where a global alignment score of a pair of two base sequences is greater than or equal to the second threshold value, it is determined that the pair of these two base sequences has high primer dimer formability, and the following step is not performed on the pair.

The second threshold value is not particularly limited and can be appropriately set. For example, the second threshold value may be set using a PCR condition such as the amount of genomic DNA which becomes a template for a polymerase chain reaction.

It is possible to set the global alignment score obtained by performing pairwise global alignment on a base sequence which has three bases and includes the 3’ terminal of a base sequence of each primer to be less than the second threshold value by setting a base sequence with several bases from the 3’ terminal of a primer as an identical base sequence.

Here, in the example in which (e) Global Alignment Step is shown, a case where the second threshold value is set to "3" is considered.

In the above-described example, the global alignment score is "-3" and is less than "3" which is the second threshold value. Therefore, it is possible to determine that the pair of the base sequences of SEQ ID No: 1 and SEQ ID No: 2 has low primer dimer formability.

The present step is performed on all of the pairs for which scores are calculated in (e) Global Alignment Step.

Both steps of (c) Local Alignment Step and (d) First Stage Selection Step are performed before or after both steps of (e) Global Alignment Step and (f) Second Stage Selection Step, or are performed in parallel with both steps of (e) Global Alignment Step and (f) Second Stage Selection Step.

In addition, in order to reduce the amount of calculation, it is preferable to perform both steps of (c) Local Alignment Step and (d) First Stage Selection Step in a combination which has passed (f) Second Stage Selection Step after first performing both steps of (e) Global Alignment Step and (f) Second Stage Selection Step. Particularly, as the number of target loci and the number of base sequences of primer candidates are increased, the effect of reducing the amount of calculation is increased, and it is possible to speed up the overall processing.

This is because the amount of calculation of a global alignment score is smaller than that of a local alignment score which is obtained by searching a partial sequence with high complementarity from the entire base sequence under the condition that the base sequence includes the 3' terminal and it is possible to speed up the processing since global alignment is performed on a base sequence with a short length, i.e. a sequence length of three bases, in (e) Global Alignment Step. It is known that the global alignment is faster than the local alignment in a case of alignment with respect to a sequence having an identical length in a well-known algorithm.

(Amplification Sequence Length-Checking Step)

As desired, an amplification sequence length-checking step may be performed.

The amplification sequence length-checking step is a step of calculating the distance between ends of base sequences of primer candidates for which it has been determined that formability of a primer dimer is low in (d) First Stage Selection Step and (f) Second Stage Selection Step, on genomic DNA or chromosomal DNA regarding pairs of the base sequences of the primer candidates, and determining whether the distance is within a predetermined range may be performed.

In a case where the distance between the ends of the base sequences is within the predetermined range, it is possible to determine that there is a high possibility that the pairs of the base sequences of the primer candidates can appropriately amplify a target locus. The distance between the ends of the base sequences of the primer candidates is not particularly limited, and can be appropriately set in accordance with the PCR condition such as the type of enzyme (DNA polymerase). For example, the distance between the ends of the base sequences of the primer candidates can be set to be within various ranges such as a range of 100 to 200 bases (pair), a range of 120 to 180 bases (pair), a range of 140 to 180 bases (pair) a range of 140 to 160 bases (pair), and a range of 160 to 180 bases (pair).

(g) Primer Employment Step

(g) Primer Employment Step is shown in the block diagram of Fig. 1 as "n-th PRIMER EMPLOYMENT STEP".

The primer employment step is a step of employing a base sequence of a base sequence of a primer candidate which has been selected in both of (d) First Stage Selection Step and (f) Second Stage Selection Step, as a base sequence of a primer for amplifying the above-described target locus.

That is, in the present step, a base sequence of a primer candidate, in which a local alignment score obtained by performing pairwise local alignment on a base sequence of each primer candidate under a condition that a partial sequence to be subjected to comparison includes the 3' terminal of the base sequence is less than the first threshold value, and a global alignment score obtained by performing pairwise global alignment on a base sequence which has a predetermined number of bases and includes the 3' terminal of the base sequence of each primer candidate is less than the second threshold value, is employed as a base sequence of a primer for amplifying a target locus.

For example, it is considered that base sequences of SEQ ID No: 1 and SEQ ID No: 2 shown in Table 7 are employed as base sequences of primers for amplifying a target locus.

[Table 7]

| | Base sequence (5' → 3') |
|---|---|
| SEQ ID No: 1: | CGCTCTTCCGATCTCTGCTTCGATGCGGACCTTCTGG |
| SEQ ID No: 2: | CGCTCTTCCGATCTGACTCTCCCACATCCGGCTATGG |

As already described, the local alignment score is "-8" and is less than "3" which is the first threshold value. Moreover, the global alignment score is "-3" and is less than "3" which is the second threshold value.

Accordingly, it is possible to employ the base sequence of the primer candidate represented by SEQ ID No: 1 and the base sequence of primer candidate represented by SEQ ID No: 2 as base sequences of primers for amplifying a target locus.

A specific embodiment (second embodiment) of the method for designing primer sets which is not part of the present invention includes, generally, the following steps:

($a_n$) an n-th target locus selection step of selecting an n-th target locus, in which primer sets used in multiplex PCR are designed, from a plurality of loci;

($b_n$) an n-th primer candidate base sequence generation step of generating at least one base sequence of a primer candidate for amplifying the n-th target locus regarding each of a forward-side primer and a reverse-side primer based on a base sequence in a vicinity region of the n-th target locus on the chromosomes;
($c_n$) an n-th local alignment step of obtaining a local alignment score by performing pairwise local alignment on the base sequence of the primer candidate for amplifying the n-th target locus under a condition that a partial sequence to be subjected to comparison includes the 3' terminal of the base sequence of the primer candidate for amplifying the n-th target locus;
($d_n$) an n-th step of first stage selection of performing first stage selection of the base sequence of the primer candidate for amplifying the n-th target locus based on the local alignment score obtained in the n-th local alignment step;
($e_n$) a n-th global alignment step of obtaining a global alignment score by performing pairwise global alignment on a base sequence which has a sequence length of three bases and includes the 3' terminal of the base sequence of the primer candidate for amplifying the n-th target locus;
($f_n$) an n-th step of second stage selection of performing second stage selection of the base sequence of the primer candidate for amplifying the n-th target locus based on the global alignment score obtained in the n-th global alignment step; and
($g_n$) an n-th primer employment step of employing the base sequence of the primer candidate which has been selected in both of the n-th step of first stage selection and the n-th step of second stage selection as a base sequence of a primer for amplifying the n-th target locus.

Here, n is an integer satisfying $n \geq 1$, and both steps of ($c_n$) n-th Local Alignment Step and ($d_n$) n-th Step of First Stage Selection are performed before or after both steps of ($e_n$) n-th Global Alignment Step and ($f_n$) n-th Step of Second Stage Selection or are performed in parallel with both steps of ($e_n$) n-th Global Alignment Step and ($f_n$) n-th Step of Second Stage Selection.
In addition, in a case where the number N (N is an integer satisfying $N \geq 2$) of a plurality of loci is greater than n, the above-described n is replaced with n + 1, and steps are repeated until primer sets are employed for all of the plurality of loci.
The steps in the case where n is replaced with n + 1 are shown below.

($a_{n+1}$) An (n + 1)th target locus selection step of selecting an (n + 1)th target locus, which is different from the already selected target locus and in which primer sets used in multiplex PCR are designed, from the plurality of loci;
($b_{n+1}$) an (n + 1)th primer candidate base sequence generation step of generating at least one base sequence of a primer candidate for amplifying the (n + 1)th target locus regarding each of a forward-side primer and a reverse-side primer based on a base sequence in a vicinity region of the (n + 1)th target locus on the chromosomes;
($c_{n+1}$) an (n + 1)th local alignment step of obtaining a local alignment score by performing pairwise local alignment on the base sequence of the primer candidate for amplifying the (n + 1)th target locus and the base sequence of the primer which has already been employed, under a condition that partial sequences to be subjected to comparison include the 3' terminal of the base sequence of the primer candidate for amplifying the (n + 1)th target locus and the 3' terminal of the base sequence of the primer which has already been employed;
($d_{n+1}$) an (n + 1)th step of first stage selection of performing first stage selection of the base sequence of the primer candidate for amplifying the (n + 1)th target locus based on the local alignment score obtained in the (n + 1)th local alignment step;
($e_{n+1}$) an (n + 1)th global alignment step of obtaining a global alignment score by performing pairwise global alignment on base sequences which have a sequence length of three bases and include the 3' terminal of the base sequence of the primer candidate for amplifying the (n + 1)th target locus and the 3' terminal of the base sequence of the primer which has already been employed;
($f_{n+1}$) an (n + 1)th step of (n + 1)th stage selection of performing (n + 1)th stage selection of the base sequence of the primer candidate for amplifying the (n + 1)th target locus based on the global alignment score obtained in the (n + 1)th global alignment step; and
($g_{n+1}$) an (n + 1)th primer employment step of employing the base sequence of the primer candidate which has been selected in both of the (n + 1)th step of first stage selection and the (n + 1)th step of (n + 1)th stage selection as a base sequence of a primer for amplifying the (n + 1)th target locus.

Here, both steps of ($c_{n+1}$) (n + 1)th Local Alignment Step and ($d_{n+1}$) (n + 1)th Step of First Stage Selection are performed before or after both steps of ($e_{n+1}$) (n + 1)th Global Alignment Step and ($f_{n+1}$) (n + 1)th Step of (n + 1)th Stage Selection, or are performed in parallel with both steps of ($e_{n+1}$) (n + 1)th Global Alignment Step and ($f_{n+1}$ ) (n + 1)th Step of (n + 1)th Stage Selection.
Each step of the second embodiment of the method for designing primer sets will be described in detail.

($a_n$) n-th Target Locus Selection Step

($a_n$) n-th Target Locus Selection Step is shown in the block diagram of Fig. 1 as "n-th TARGET LOCUS SELECTION STEP".
($a_n$) n-th Target Locus Selection Step is the same as "(a) Target Locus Selection Step" of the first embodiment except that an n-th target locus is selected.

However, in a case where $n \geq 2$, a locus different from the target locus selected up to an (n-1)th target locus selection step is selected.
In a case where $n \geq 2$, the selection of the n-th target locus can be simultaneously performed with the selection of an (n-1)th target locus, or can be performed after the selection of the (n-1)th target locus.

($b_n$) n-th Primer Candidate Base Sequence Generation Step

($b_n$) n-th Primer Candidate Base Sequence Generation Step is shown in the block diagram of Fig. 1 as "n-th PRIMER CANDIDATE BASE SEQUENCE GENERATION STEP".
($b_n$) n-th Primer Candidate Base Sequence Generation Step is the same as "(b) Primer Candidate Base Sequence Generation Step" of the first embodiment of the method for designing primer sets in the present invention except that the base sequence of the primer candidate for amplifying the n-th target locus is generated.

(Specificity-Checking Step)

The specificity-checking step is the same as "Specificity-Checking Step" of the first embodiment of the method for designing primer sets in the present invention. The present step is an arbitrary step, and may be performed or may not be performed.

($c_n$) n-th Local Alignment Step

($c_n$) n-th Local Alignment Step is shown in the block diagram of Fig. 1 as "n-th LOCAL ALIGNMENT STEP".
($c_n$) n-th Local Alignment Step is the same as "(c) Local Alignment Step" of the first embodiment of the method for designing primer sets in the present invention except that local alignment is performed on the base sequence of the primer candidate for amplifying the n-th target locus generated in ($b_n$) n-th Primer Candidate Base Sequence Generation Step.

However, in a case where $n \geq 2$, local alignment is performed on the base sequence of the primer candidate for amplifying the n-th target locus generated in ($b_n$) n-th Primer Candidate Base Sequence Generation Step and base sequences of primers which have already been employed. Here, all the base sequences of the primers which have already been employed are base sequences which have been employed as base sequences of primers for amplifying target loci from the first target locus to the (n-1)th target locus (the same applies hereinafter).

($d_n$) n-th Step of First Stage Selection

($d_n$) n-th Step of First Stage Selection is shown in the block diagram of Fig. 1 as "n-th STEP OF FIRST STAGE SELECTION".
($d_n$) n-th Step of First Stage Selection is the same as "(d) First Stage Selection Step" of the first embodiment of the method for designing primer sets in the present invention except that the selection is performed on the base sequence of the primer candidate for amplifying the n-th target locus generated in ($b_n$) n-th Primer Candidate Base Sequence Generation Step, based on the local alignment score obtained in ($c_n$) n-th Local Alignment Step.

However, in a case where $n \geq 2$, selection is performed on the base sequence of the primer candidate for amplifying the n-th target locus generated in ($b_n$) n-th Primer Candidate Base Sequence Generation Step and base sequences of primers which have already been employed.

($e_n$) n-th Global Alignment Step

($e_n$) n-th Global Alignment Step is shown in the block diagram of Fig. 1 as "n-th GLOBAL ALIGNMENT STEP". ($e_n$) n-th Global Alignment Step is the same as "(e) Global Alignment Step" of the first embodiment of the method for designing primer sets in the present invention except that global alignment is performed on the base sequence of the primer candidate for amplifying the n-th target locus generated in ($b_n$) n-th Primer Candidate Base Sequence Generation Step.

However, in a case where $n \geq 2$, global alignment is performed on the base sequence of the primer candidate for amplifying the n-th target locus generated in ($b_n$) n-th Primer Candidate Base Sequence Generation Step and base sequences of primers which have already been employed.

($f_n$) n-th Step of Second Stage Selection

($f_n$) n-th Step of Second Stage Selection is shown in the block diagram of Fig. 1 as "n-th STEP OF SECOND STAGE SELECTION".

($f_n$) n-th Step of Second Stage Selection is the same as "(f) Second Stage Selection Step" of the first embodiment of the method for designing primer sets in the present invention except that the selection is performed on the base sequence of the primer candidate for amplifying the n-th target locus generated in ($b_n$) n-th Primer Candidate Base Sequence Generation Step, based on the global alignment score obtained in ($e_n$) n-th Global Alignment Step.

However, in a case where $n \geq 2$, selection is performed on the base sequence of the primer candidate for amplifying the n-th target locus generated in ($b_n$) n-th Primer Candidate Base Sequence Generation Step and base sequences of primers which have already been employed.

Similarly to the first embodiment of the method for designing primer sets in the present invention, both steps of ($c_n$) n-th Local Alignment Step and ($d_n$) n-th Step of First Stage Selection are performed before or after both steps of ($e_n$) n-th Global Alignment Step and ($f_n$) n-th Step of Second Stage Selection, or are performed in parallel with both steps of ($e_n$) n-th Global Alignment Step and ($f_n$) n-th Step of Second Stage Selection.

In addition, in order to reduce the amount of calculation, it is preferable to perform both steps of ($c_n$) n-th Local Alignment Step and ($d_n$) n-th Step of First Stage Selection in a combination which has passed ($f_n$) n-th Step of Second Stage Selection after performing both steps of ($e_n$) n-th Global Alignment Step and ($f_n$) n-th Step of Second Stage Selection" first. Particularly, as the number of target loci and the number of base sequences of primer candidates are increased, the effect of reducing the amount of calculation is increased, and it is possible to speed up the overall processing.

(Amplification Sequence Length-Checking Step)

The specificity-checking step is the same as "Amplification Sequence Length-Checking Step" of the first embodiment of the method for designing primer sets in the present invention. The present step is an arbitrary step, and may be performed or may not be performed.

($g_n$) n-th Primer Employment Step

($g_n$) n-th Primer Employment Step is shown in the block diagram of Fig. 1 as "n-th PRIMER EMPLOYMENT STEP". ($g_n$) n-th Primer Employment Step is the same as "(g) Primer Employment Step" of the first embodiment of the method for designing primer sets in the present invention.

Hereinafter, the present invention will be described in more detail using an example, but is not limited to these examples.

Examples

[Single Cell Isolation and Genomic DNA Extraction]

<Single Cell Isolation>

(Acquisition of Peripheral Blood Sample)

10.5 mg of sodium salts of ethylenediaminetetraacetic acid (EDTA) was added to a 7 mL blood collecting tube as an anticoagulant, and then, 7 mL of peripheral blood was obtained within the blood collecting tube as volunteer blood after obtaining informed consent from a pregnant woman volunteer. Thereafter, the blood was diluted using physiological salt solution.

(Concentration of Nucleated Red Blood Cell)

A liquid with a density of 1.070 (g/cm$^3$) and a liquid with a density of 1.095 (g/cm$^3$) were prepared using PERCOLL LIQUID (manufactured by GE Healthcare Bioscience), 2 mL of a liquid with a density of 1.095 g/mL was added to the bottom portion of a centrifuge tube, and the centrifuge tube was cooled in a refrigerator for 30 minutes at 4°C.

Thereafter, the centrifuge tube was taken out from the refrigerator and 2 mL of a liquid with a density of 1.070 (g/cm$^3$) was made to slowly overlap the top of the liquid with a density of 1.095 (g/cm$^3$) so as not to disturb the interface.

Then, 11 mL of diluent of blood which had been collected above was slowly added to the top of the medium with a density of 1.070 (g/cm$^3$) in the centrifuge tube.

Thereafter, centrifugation was performed for 20 minutes at 2,000 rpm.

The centrifuge tube was taken out and fractions which had been deposited between the liquid with a density of 1.070 (g/cm$^3$) and the liquid with a density of 1.095 (g/cm$^3$) were collected using a pipette.

A droplet of the fractions of blood which have been collected in this manner was spotted at one end of a slide glass substrate 1 while holding the slide glass substrate 1 using one hand. A slide glass substrate 2 was held by the other hand and one end of the slide glass substrate 2 was brought into contact with the slide glass substrate 1 at an angle of 30°. The contact surface of the slide glass substrate 2 which was brought into contact with the fractions of blood was then spread into the space surrounded by the two sheets of slide glass due to a capillary phenomenon.

Next, the slide glass substrate 2 was made to be slid in a direction of a region opposite to the region of the slide glass substrate 1, on which blood was placed, while maintaining the angle, and the slide glass substrate 1 was uniformly coated with blood. After the completion of coating, the slide glass substrate 1 was sufficiently dried through air blowing for one or more hours. This glass substrate was immersed in a MAY-Grunwald staining liquid for 3 minutes and was washed by being immersed in a phosphoric acid buffer solution. Thereafter, the glass substrate was immersed in a GIEMSA staining liquid, which was diluted with a phosphoric acid buffer solution to make a concentration of 3%, for 10 minutes.

Thereafter, a plurality of stained glass substrates were prepared by being dried after being washed with pure water.

(Identification of Nucleated Red Blood Cell Using information on Shape of Cell)

In order to sort out nucleated red blood cell candidates from the cells with which the top of the slide glass substrate was coated, a measurement system of an optical microscope provided with an electric XY stage, an objective lens, and a CCD camera, a control unit provided with an XY stage control unit and a Z-direction control unit, and a control unit portion including an image input unit, an image processing unit, and an XY position recording unit were prepared. Blood cells which had been prepared as described above and with which the top of the slide glass substrate was coated were placed on the XY stage and scanning was performed by performing focusing on the slide glass. An image which was obtained using an optical microscope was taken and nucleated red blood cells which were objective cells were searched through image analysis.

In the image analysis, cells which satisfied the two following conditions were detected and the XY position was recorded.

$$0.25 < N / C < 1.0 \cdots (1)$$

$$0.65 < N / L^2 < 0.785 \cdots (2)$$

Here, "N" represents the area of a nuclear region of a cell on which image analysis is to be performed, "C" represents the area of cytoplasm of a cell on which image analysis is to be performed, and "L" represents the length of the major axis of a nucleus of a cell on which image analysis is to be performed. The length of the major axis of a nucleus of a cell is defined as a length of the major axis of an elliptical shape circumscribing a cell nucleus which has a complicated shape.

Nucleated red blood cells which satisfy Formulas (1) and (2) were selected from nucleated red blood cells existing on the slide glass substrate, and were regarded as nucleated red blood cell candidates of the next step.

(Sorting of Fetal Nucleated Red Blood Cell)

Analysis of spectral information was performed on the nucleated red blood cell candidates, which had been identified in the step of identifying nucleated red blood cells using information on the shape of cells, using a micro spectrometer.

The nucleated red blood cell candidates on the slide glass substrate were specified, one cell among them was irradiated with monochromatic light in the vicinity of 415 nm, and the absorption coefficient of the cell was measured.

Next, three white blood cells of which the shapes of nuclei in the vicinity of the cell did not satisfy Formula (2) were selected from cells closest to the nucleated red blood cell candidates. The absorption coefficient of each white blood cell was calculated in the same manner, and an average absorption coefficient was calculated.

The absorption coefficients of remaining cells of the nucleated red blood cell candidates on the slide glass substrate were also measured similarly to the above, and an average value of the absorption coefficients of three white blood cells in the vicinity of each cell was calculated. Cells of which the ratio of the absorption coefficient of a nucleated red blood cell candidate to the average absorption coefficient of the white blood cells becomes greater than or equal to 1 were extracted from these results. As a result, 8 cells of which the ratio was clearly greater than or equal to 1 were detected.

(Cell Collection)

The 8 cells determined as described above were collected using a micromanipulator.

<Genomic DNA Extraction>

Cytolysis was performed on the collected single cells using a Single Cell WGA kit (manufactured by New England Biolabs). That is, each of the single cells was mixed in 5 μL of Cell extraction buffer, 4.8 μL of Extraction Enzyme Dilution Buffer and 0.2 μL of Cell Extraction Enzyme were mixed with the mixture to make the total amount of the solution be 10 μL, the solution was incubated for 10 minutes at 75°C, and then, the solution was further incubated for 4 minutes at 95°C, in accordance with the description of "Sample Preparation Methods" and "Pre-Amplification Protocol" in an instruction attached to the kit.

Accordingly, genomic DNA was prepared.

[Example 1]

<Selection of Locus to be Amplified through Multiplex PCR>

Chromosomes of interest are chromosome 13, chromosome 18, and chromosome 21. Regarding the number of loci to be amplified through multiplex PCR, the number of loci on chromosome 13 was set to 181, the number of loci on chromosome 18 was set to 178, the number of loci on chromosome 21 was set to 188, the number of loci on an X chromosome was set to 51, and the number of loci on a Y chromosome was set to 49 so that the total number of loci became 647. The selected loci are shown in Tables 8 to 12.

[Table 8]

| Loci (coordinate) on chromosome 13 | | | | |
|---|---|---|---|---|
| chr13: 19751127 | chr13: 28609723 | chr13: 39454452 | chr13: 76055820 | chr13: 111134858 |
| chr13: 19751657 | chr13: 28636084 | chr13: 39587572 | chr13: 76395620 | chr13: 111154058 |

(continued)

| Loci (coordinate) on chromosome 13 | | | | |
|---|---|---|---|---|
| chr13: 20763113 | chr13: 28893642 | chr13: 41134003 | chr13: 76397731 | chr13: 111155773 |
| chr13: 20763380 | chr13: 29599322 | chr13: 41134396 | chr13: 76423248 | chr13: 111287047 |
| chr13: 21006355 | chr13: 29599723 | chr13: 41323397 | chr13: 76432051 | chr13: 111293915 |
| chr13: 21205192 | chr13: 29600415 | chr13: 41379272 | chr13: 77570078 | chr13: 111932927 |
| chr13: 21553872 | chr13: 29608252 | chr13: 41508049 | chr13: 77632470 | chr13: 111992247 |
| chr13: 21555696 | chr13: 29855847 | chr13: 41515368 | chr13: 84455178 | chr13: 113053470 |
| chr13: 21620085 | chr13: 30097543 | chr13: 41533052 | chr13: 86369981 | chr13: 113210444 |
| chr13: 22255230 | chr13: 31318222 | chr13: 41808035 | chr13: 88328960 | chr13: 113473629 |
| chr13: 23894812 | chr13: 31338174 | chr13: 41814520 | chr13: 95858978 | chr13: 113512574 |
| chr13: 23898509 | chr13: 31711623 | chr13: 42872719 | chr13: 96205154 | chr13: 113532554 |
| chr13: 23904976 | chr13: 32360511 | chr13: 43930140 | chr13: 96258288 | chr13: 113719264 |
| chr13: 23913017 | chr13: 32785086 | chr13: 44457925 | chr13: 98828892 | chr13: 113728781 |
| chr13: 24243004 | chr13: 33628138 | chr13: 45147990 | chr13: 98865546 | chr13: 113770068 |
| chr13: 24432997 | chr13: 33635835 | chr13: 45149662 | chr13: 99030075 | chr13: 113801737 |
| chr13: 24797383 | chr13: 33684151 | chr13: 46115731 | chr13: 99037076 | chr13: 113960845 |
| chr13: 24797913 | chr13: 33704065 | chr13: 46124375 | chr13: 99100547 | chr13: 114088080 |
| chr13: 24823699 | chr13: 35517239 | chr13: 46541673 | chr13: 99337039 | chr13: 114098849 |
| chr13: 24860985 | chr13: 36348767 | chr13: 46638826 | chr13: 99356611 | chr13: 114150007 |
| chr13: 24895805 | chr13: 36382373 | chr13: 46648094 | chr13: 99364165 | chr13: 114154419 |
| chr13: 25009099 | chr13: 36385031 | chr13: 46946157 | chr13: 99447005 | chr13: 114307200 |
| chr13: 25029218 | chr13: 36686138 | chr13: 49070345 | chr13: 99449468 | chr13: 114307693 |
| chr13: 25265103 | chr13: 36801415 | chr13: 50126382 | chr13: 99449717 | chr13: 114309226 |
| chr13: 25266932 | chr13: 36886469 | chr13: 50134099 | chr13: 99457431 | chr13: 114323997 |
| chr13: 25280464 | chr13: 37679333 | chr13: 50204880 | chr13: 99907341 | chr13: 114325855 |
| chr13: 25281181 | chr13: 38266328 | chr13: 50589718 | chr13: 99948097 | chr13: 114514771 |
| chr13: 25453420 | chr13: 38924062 | chr13: 51918558 | chr13: 101736075 | chr13: 114762198 |
| chr13: 25671755 | chr13: 39262057 | chr13: 52313195 | chr13: 102366825 | chr13: 115090193 |
| chr13: 25743748 | chr13: 39262435 | chr13: 52348164 | chr13: 103389420 | |
| chr13: 26043182 | chr13: 39263023 | chr13: 52518383 | chr13: 103396716 | |
| chr13: 26144896 | chr13: 39263714 | chr13: 52971893 | chr13: 103397030 | |
| chr13: 26620924 | chr13: 39263961 | chr13: 53624380 | chr13: 103402099 | |
| chr13: 27256807 | chr13: 39265511 | chr13: 60240961 | chr13: 103528002 | |
| chr13: 27333412 | chr13: 39266138 | chr13: 61986918 | chr13: 108518444 | |
| chr13: 27845643 | chr13: 39266565 | chr13: 67800935 | chr13: 109496813 | |
| chr13: 28367956 | chr13: 39424253 | chr13: 67802095 | chr13: 110833702 | |
| chr13: 28537317 | chr13: 39438560 | chr13: 67802513 | chr13: 111077197 | |

[Table 9]

| Loci (coordinate) on chromosome 18 | | | | |
|---|---|---|---|---|
| chr18: 346821 | chr18: 10705744 | chr18: 29164577 | chr18: 44149474 | chr18: 61233861 |
| chr18: 2890768 | chr18: 10706794 | chr18: 29178549 | chr18: 44470706 | chr18: 61264298 |
| chr18: 2892188 | chr18: 10714830 | chr18: 29784171 | chr18: 44560429 | chr18: 61379825 |
| chr18: 2914310 | chr18: 10726909 | chr18: 29848436 | chr18: 44561619 | chr18: 61390316 |
| chr18: 2921592 | chr18: 10763007 | chr18: 29855491 | chr18: 44589450 | chr18: 61650896 |
| chr18: 2922149 | chr18: 10800448 | chr18: 29867174 | chr18: 44626630 | chr18: 65181049 |
| chr18: 2940811 | chr18: 11071481 | chr18: 29867688 | chr18: 44627245 | chr18: 66504093 |
| chr18: 3071878 | chr18: 11884567 | chr18: 32156889 | chr18: 47017820 | chr18: 71816278 |
| chr18: 3075712 | chr18: 12292025 | chr18: 32919934 | chr18: 47369758 | chr18: 72103918 |

(continued)

| Loci (coordinate) on chromosome 18 | | | | |
|---|---|---|---|---|
| chr18: 3115877 | chr18: 12971179 | chr18: 33552862 | chr18: 47414638 | chr18: 72109211 |
| chr18: 3129399 | chr18: 13056333 | chr18: 33750046 | chr18: 47489410 | chr18: 72593032 |
| chr18: 3151695 | chr18: 13059173 | chr18: 33779855 | chr18: 47500836 | chr18: 72897316 |
| chr18: 3188976 | chr18: 19153494 | chr18: 33831189 | chr18: 47511113 | chr18: 72998703 |
| chr18: 3457539 | chr18: 20951443 | chr18: 34162836 | chr18: 47563299 | chr18: 72999000 |
| chr18: 3702419 | chr18: 21100240 | chr18: 34273228 | chr18: 47751130 | chr18: 72999819 |
| chr18: 5416160 | chr18: 21122781 | chr18: 34311363 | chr18: 47777937 | chr18: 74153252 |
| chr18: 5551142 | chr18: 21136274 | chr18: 34324091 | chr18: 47800179 | chr18: 74274762 |
| chr18: 5956238 | chr18: 21140432 | chr18: 34378445 | chr18: 47803354 | chr18: 74639368 |
| chr18: 6943264 | chr18: 21229102 | chr18: 34850846 | chr18: 48190412 | chr18: 74671768 |
| chr18: 6965340 | chr18: 21390826 | chr18: 39647381 | chr18: 48327815 | chr18: 74680259 |
| chr18: 6973197 | chr18: 21413869 | chr18: 42529996 | chr18: 48703073 | chr18: 74830377 |
| chr18: 6977844 | chr18: 21441717 | chr18: 42530796 | chr18: 54814730 | chr18: 74980601 |
| chr18: 6983194 | chr18: 21481203 | chr18: 42531834 | chr18: 55020359 | chr18: 77227476 |
| chr18: 6986227 | chr18: 21485200 | chr18: 42532606 | chr18: 55143766 | chr18: 77287531 |
| chr18: 6999665 | chr18: 21489153 | chr18: 42533130 | chr18: 55247336 | chr18: 77805778 |
| chr18: 7888198 | chr18: 21496554 | chr18: 43206985 | chr18: 55317591 | chr18: 77894844 |
| chr18: 7955213 | chr18: 22056766 | chr18: 43219877 | chr18: 55998093 | |
| chr18: 8379296 | chr18: 22804549 | chr18: 43258936 | chr18: 56067804 | |
| chr18: 8387065 | chr18: 22805428 | chr18: 43432600 | chr18: 56137685 | |
| chr18: 8783835 | chr18: 22807059 | chr18: 43490602 | chr18: 56184191 | |
| chr18: 8790019 | chr18: 23866349 | chr18: 43491853 | chr18: 56202982 | |
| chr18: 8796223 | chr18: 24126875 | chr18: 43492274 | chr18: 56246845 | |
| chr18: 8798185 | chr18: 24497240 | chr18: 43495660 | chr18: 59195354 | |
| chr18: 8803625 | chr18: 25543387 | chr18: 43496165 | chr18: 60027241 | |
| chr18: 8806945 | chr18: 28649042 | chr18: 44087565 | chr18: 60053990 | |
| chr18: 10485680 | chr18: 28934681 | chr18: 44098220 | chr18: 60241732 | |
| chr18: 10699017 | chr18: 28956904 | chr18: 44104697 | chr18: 60812027 | |
| chr18: 10699888 | chr18: 29122799 | chr18: 44114380 | chr18: 61154206 | |

[Table 10]

| Loci (coordinate) on chromosome 21 | | | | |
|---|---|---|---|---|
| chr21: 15882622 | chr21: 33867447 | chr21: 40338115 | chr21: 43783418 | chr21: 46902703 |
| chr21: 16340289 | chr21: 33881034 | chr21: 40777873 | chr21: 43792869 | chr21: 46924383 |
| chr21: 19713821 | chr21: 33921989 | chr21: 40873485 | chr21: 43805637 | chr21: 47243535 |
| chr21: 19756050 | chr21: 33962803 | chr21: 41032740 | chr21: 43807322 | chr21: 47296634 |
| chr21: 27284122 | chr21: 33976489 | chr21: 41295995 | chr21: 43808526 | chr21: 47349899 |
| chr21: 27372388 | chr21: 34072139 | chr21: 41361796 | chr21: 43846762 | chr21: 47351282 |
| chr21: 27394285 | chr21: 34619143 | chr21: 41559182 | chr21: 43852232 | chr21: 47355726 |
| chr21: 27451555 | chr21: 34634991 | chr21: 41621714 | chr21: 43853775 | chr21: 47361589 |
| chr21: 27852641 | chr21: 34749253 | chr21: 41652905 | chr21: 43856895 | chr21: 47404302 |
| chr21: 28122730 | chr21: 34787312 | chr21: 41684090 | chr21: 43864694 | chr21: 47544599 |
| chr21: 28212760 | chr21: 34975846 | chr21: 42812891 | chr21: 43867288 | chr21: 47545482 |
| chr21: 28214910 | chr21: 35237608 | chr21: 42813714 | chr21: 43873037 | chr21: 47614443 |
| chr21: 28305212 | chr21: 35260481 | chr21: 42817937 | chr21: 43913135 | chr21: 47627429 |
| chr21: 30316850 | chr21: 35467645 | chr21: 43032387 | chr21: 43954936 | chr21: 47632006 |
| chr21: 30342933 | chr21: 35721596 | chr21: 43161103 | chr21: 44180443 | chr21: 47636469 |
| chr21: 30365162 | chr21: 35757862 | chr21: 43162206 | chr21: 44189166 | chr21: 47639492 |

(continued)

| Loci (coordinate) on chromosome 21 | | | | |
|---|---|---|---|---|
| chr21: 30408670 | chr21: 37233883 | chr21: 43169357 | chr21: 44306874 | chr21: 47639800 |
| chr21: 30464866 | chr21: 37444822 | chr21: 43221797 | chr21: 44323720 | chr21: 47641794 |
| chr21: 31587677 | chr21: 37444973 | chr21: 43242946 | chr21: 44324365 | chr21: 47660086 |
| chr21: 31691792 | chr21: 37518706 | chr21: 43255625 | chr21: 44473980 | chr21: 47662759 |
| chr21: 31692173 | chr21: 37610969 | chr21: 43325863 | chr21: 45211298 | chr21: 47704896 |
| chr21: 31709691 | chr21: 37612139 | chr21: 43327117 | chr21: 45217905 | chr21: 47775389 |
| chr21: 31812772 | chr21: 37617575 | chr21: 43412853 | chr21: 45379986 | chr21: 47776780 |
| chr21: 31866472 | chr21: 37618190 | chr21: 43424851 | chr21: 45542193 | chr21: 47782264 |
| chr21: 31874211 | chr21: 37752637 | chr21: 43436743 | chr21: 45656774 | chr21: 47786494 |
| chr21: 32201822 | chr21: 37833407 | chr21: 43509956 | chr21: 45713737 | chr21: 47811272 |
| chr21: 32253513 | chr21: 37904343 | chr21: 43510437 | chr21: 45732116 | chr21: 47836206 |
| chr21: 32638549 | chr21: 37975323 | chr21: 43514676 | chr21: 45738376 | hr21: 47851777 |
| chr21: 32853499 | chr21: 38117505 | chr21: 43519032 | chr21: 45876620 | chr21: 47954017 |
| chr21: 33068525 | chr21: 38285420 | chr21: 43520551 | chr21: 45987736 | chr21: 47954427 |
| chr21: 33340639 | chr21: 38309459 | chr21: 43522349 | chr21: 46032094 | hr21: 47957354 |
| chr21: 33346936 | chr21: 38439597 | chr21: 43523594 | chr21: 46057391 | chr21: 47966843 |
| chr21: 33371123 | chr21: 38568009 | chr21: 43524018 | chr21: 46313442 | chr21: 47969793 |
| chr21: 33678976 | chr21: 38600557 | chr21: 43546509 | chr21: 46320313 | chr21: 47975686 |
| chr21: 33691710 | chr21: 39671476 | chr21: 43704683 | chr21: 46600355 | chr21 47977678 |
| chr21: 33694120 | chr21: 39930986 | chr21: 43705994 | chr21: 46612428 | chr21: 47985655 |
| chr21: 33719343 | chr21: 40190443 | chr21: 43708041 | chr21: 46624583 | |
| chr21: 33755763 | chr21: 40191431 | chr21: 43764586 | chr21: 46900410 | |

[Table 11]

| Loci (coordinate) on X chromosome | | | |
|---|---|---|---|
| chrX: 2779570 | chrX: 46472826 | chrX: 108708552 | chrX:153051907 |
| chrX: 2951434 | chrX: 48418126 | chrX: 112024157 | chrX:153070999 |
| chrX: 3241050 | chrX: 49061742 | chrX: 117527020 | chrX: 153132261 |
| chrX: 6995417 | chrX: 50130544 | chrX: 118219347 | chrX:153171993 |
| chrX: 14027177 | chrX: 50659280 | chrX:122537277 | chrX:153219665 |
| chrX: 16168467 | chrX: 53577887 | chrX:125955199 | chrX: 153633359 |
| chrX: 16627756 | chrX: 69250308 | chrX: 134483151 | |
| chrX: 17746244 | chrX: 69261818 | chrX: 134991078 | |
| chrX: 19375782 | chrX: 69478749 | chrX: 135593337 | |
| chrX: 22291606 | chrX: 69572490 | chrX: 136112707 | |
| chrX: 26157220 | chrX: 69749852 | chrX:141290865 | |
| chrX: 27839572 | chrX: 69890301 | chrX:151129822 | |
| chrX: 30261002 | chrX: 70146475 | chrX: 152226542 | |
| chrX: 39932907 | chrX: 84363140 | chrX: 153048403 | |
| chrX: 43603391 | chrX: 96139406 | chrX: 153049739 | |

[Table 12]

| Loci (coordinate) on Y chromosome | | | |
|---|---|---|---|
| chrY: 2710802 | chrY: 9878799 | chrY: 16963396 | chrY: 23297891 |

(continued)

| Loci (coordinate) on Y chromosome | | | |
|---|---|---|---|
| chrY: 2837564 | chrY: 10038191 | chrY: 17111947 | chrY: 23370399 |
| chrY: 6740540 | chrY: 13209049 | chrY: 17115626 | chrY: 23428429 |
| chrY: 6946990 | chrY: 13864997 | chrY: 17358415 | chrY: 24377754 |
| chrY: 7229757 | chrY: 14082469 | chrY: 17753399 | |
| chrY: 7262370 | chrY: 14655058 | chrY: 17833136 | |
| chrY: 7526916 | chrY: 14705721 | chrY: 18811624 | |
| chrY: 7679062 | chrY: 14756402 | chrY: 18956584 | |
| chrY: 7961690 | chrY: 14944834 | chrY: 21156181 | |
| chrY: 8113984 | chrY: 15053566 | chrY: 21906335 | |
| chrY: 8396638 | chrY: 15238478 | chrY: 21924529 | |
| chrY: 8624945 | chrY: 15716813 | chrY: 22902924 | |
| chrY: 8632095 | chrY: 15872674 | chrY: 22921056 | |
| chrY: 8892984 | chrY: 16021217 | chrY: 23232726 | |
| chrY: 9114110 | chrY: 16325632 | chrY: 23239880 | |

<Design of Primer Set>

A primer set used in multiplex PCR was designed, selected, and employed according to the above-described method for designing primer sets.

The primer names, the base sequences, and the SEQ ID Nos of 20 pairs of target loci among each of the target loci on chromosome 13, chromosome 18, chromosome 21, an X chromosome, and a Y chromosome employed as primer sets for PCR amplification are shown in Tables 13 to 17.

In a case of designing the primer sets, the size of an amplification product was set to 140 bp to 180 bp, the Tm value was set to 60°C to 70°C, and the length of a complementary portion of a primer was set to 20 mer.

Selection of primer sets was performed by calculating scores using the scoring system shown in Table 1 and setting all of the threshold values of a local alignment score and a global alignment score to "+3".

[Table 13]

| Primer set for chromosome 13 | | |
|---|---|---|
| Primer name | Base sequence (5' → 3') | SEQ ID No |
| chrl3: 20763380: Fwd | CGCTCTTCCGATCTCTGCTTCGATGCGGACCTTCTGG | 1 |
| chrl3: 20763380: Rev | CGCTCTTCCGATCTGACTCTCCCACATCCGGCTATGG | 2 |
| chrl3: 21205192: Fwd | CGCTCTTCCGATCTCTGTTTCCCCGACCATAAGCTTG | 3 |
| chrl3: 21205192: Rev | CGCTCTTCCGATCTGACATACAGGGCTGAGAGATTGG | 4 |
| chrl3: 21620085: Fwd | CGCTCTTCCGATCTCTGTGATAAGGTCCGAACTTTGG | 5 |
| chrl3 :21620085: Rev | CGCTCTTCCGATCTGACGCGACTGCAAGAGATTCGTG | 6 |
| chrl3: 23898509: Fwd | CGCTCTTCCGATCTCTGATTTGCTGCTGACCAGGGTG | 7 |
| chrl3: 23898509: Rev | CGCTCTTCCGATCTGACAGGTACAGCTTCCCATCTGG | 8 |
| chrl3: 24797913: Fwd | CGCTCTTCCGATCTCTGCCGTGTGTGAGATTCTCGTG | 9 |
| chrl3: 24797913: Rev | CGCTCTTCCGATCTGACACTGCTCAGGGTCCTCTGTG | 10 |
| chrl3: 25009099: Fwd | CGCTCTTCCGATCTCTGGTAAAGCCTCCAGGATGTTG | 11 |
| chrl3: 25009099: Rev | CGCTCTTCCGATCTGACCTGGCACTTGTGCTGACTGG | 12 |
| chrl3: 25029218: Fwd | CGCTCTTCCGATCTCTGCCAAAGCGCACTCACCTGTG | 13 |
| chrl3: 25029218: Rev | CGCTCTTCCGATCTGACTAGCCAGTGAGAGCGAAGTG | 14 |

(continued)

| Primer set for chromosome 13 | | |
|---|---|---|
| Primer name | Base sequence (5' → 3') | SEQ ID No |
| chrl3: 25265103: Fwd<br>chrl3: 25265103: Rev | CGCTCTTCCGATCTCTGGGCCTAGAGGACGATGCTTG<br>CGCTCTTCCGATCTGACTGTTGATAACCATGCCGGTG | 15<br>16 |
| chrl3: 25266932: Fwd<br>chrl3: 25266932: Rev | CGCTCTTCCGATCTCTGTGCTGGACAGTGACTCATGG<br>CGCTCTTCCGATCTGACCATTTTCCTGTCCTGGCTTG | 17<br>18 |
| chrl3: 25453420: Fwd<br>chrl3: 25453420: Rev | CGCTCTTCCGATCTCTGATCCAGTTCATATGCCGTTG<br>CGCTCTTCCGATCTGACGCGTTGCTGTCATTCCTTTG | 19<br>20 |

In addition, regarding the primer consisting of a base sequence of SEQ ID No: 1 and the primer consisting of a base sequence of SEQ ID No: 2, local alignment performed under the condition of inclusion of the 3' terminal according to the method for designing primer sets in the present invention, a local alignment score, global alignment performed on three bases of the 3' terminal of the primers according to the method for designing primer sets in the present invention, and a global alignment score are shown in Fig. 2.

As shown in Fig. 2, the base sequence of SEQ ID No: 1 and the base sequence of SEQ ID No: 2 had a local alignment score of "-8" and global alignment score of "-3", both of which were less than the set threshold value.

[Table 14]

| Primer set for chromosome 18 | | |
|---|---|---|
| Primer name | Base sequence (5' → 3') | SEQ ID No |
| chr18: 346821: Fwd<br>chr18: 346821: Rev | CGCTCTTCCGATCTCTGAGGTTCTGCTCGTTGGCTTG<br>CGCTCTTCCGATCTGACCCAAGAAGGACACGGATTGG | 21<br>22 |
| chr18: 3075712: Fwd<br>chr18: 3075712: Rev | CGCTCTTCCGATCTCTGAGCTTCCCGGGAAATTAGTG<br>CGCTCTTCCGATCTGACGAATTTTAATCGCCCCTGTG | 23<br>24 |
| chr18: 3188976: Fwd<br>chr18: 3188976: Rev | CGCTCTTCCGATCTCTGGGACTGCTTAGATGCCGTGG<br>CGCTCTTCCGATCTGACAGTCAAAAGCATGTCAGTGG | 25<br>26 |
| chr18: 3457539: Fwd<br>chr18: 3457539: Rev | CGCTCTTCCGATCTCTGCTCTGTGGAGTCCGTGATGG<br>CGCTCTTCCGATCTGACATGCAGTCACAGTGGTATGG | 27<br>28 |
| chr18: 5416160: Fwd<br>chrl8: 5416160: Rev | CGCTCTTCCGATCTCTGGGTAATGCTGCAAGCTCTGG<br>CGCTCTTCCGATCTGACCCTAGGGGATCAAGATGTGG | 29<br>30 |
| chr18: 5956238: Fwd<br>chr18: 5956238: Rev | CGCTCTTCCGATCTCTGATTCATCCCCTGACTTCTTG<br>CGCTCTTCCGATCTGACTATTTGCAGCAGATCGATGG | 31<br>32 |
| chr18: 6943264: Fwd<br>chr18: 6943264: Rev | CGCTCTTCCGATCTCTGCACCAACATAAATGGGATTG<br>CGCTCTTCCGATCTGACGCCACTGTGCTCTGTGATGG | 33<br>34 |
| chr18: 6983194: Fwd<br>chrl8: 6983194: Rev | CGCTCTTCCGATCTCTGAGTCCTGTGAGCATCTCTGG<br>CGCTCTTCCGATCTGACTGAGTGAATTGGCAAGTTTG | 35<br>36 |
| chr18: 8796223: Fwd<br>chr18: 8796223: Rev | CGCTCTTCCGATCTCTGGCAGTTTGCAGGGACTCTTG<br>CGCTCTTCCGATCTGACCACTCCAGGTTCGCTCATGG | 37<br>38 |
| chrl8: 8798185: Fwd<br>chr18: 8798185: Rev | CGCTCTTCCGATCTCTGCTCCTGCCTGTTTCAGGGTG<br>CGCTCTTCCGATCTGACAATCTGCACCCGGGAGTGTG | 39<br>40 |

In addition, regarding the primer consisting of a base sequence of SEQ ID No: 21 and the primer consisting of a base sequence of SEQ ID No: 22, local alignment performed under the condition of inclusion of the 3' terminal according to the method for designing primer sets in the present invention, a local alignment score, global alignment performed on three bases of the 3' terminal of the primers according to the method for designing primer sets in the present invention, and a global alignment score are shown in Fig. 3.

As shown in Fig. 3, the base sequence of SEQ ID No: 21 and the base sequence of SEQ ID No: 22 had a local alignment score of "-7" and global alignment score of "-3", both of which were less than the set threshold value.

[Table 15]

| Primer set for chromosome 21 | | |
|---|---|---|
| Primer name | Base sequence (5' → 3') | SEQID No |
| chr21: 16340289: Fwd | CGCTCTTCCGATCTCTGGCAACAGTCTGGCTTTTTG | 41 |
| chr21: 16340289: Rev | CGCTCTTCCGATCTGACGGGATCAGGTACTGCCGTTG | 42 |
| chr21: 28212760: Fwd | CGCTCTTCCGATCTCTGCAAGGTGCTACATGTGCTGG | 43 |
| chr21: 28212760: Rev | CGCTCTTCCGATCTGACCCTTTGCAGGGGAATGTTTG | 44 |
| chr21: 28305212: Fwd | CGCTCTTCCGATCTCTGGAGCAGCGTACCATTGGGTG | 45 |
| chr21: 28305212: Rev | CGCTCTTCCGATCTGACCAGTGTTCTCGCTCATGTGG | 46 |
| chr21: 30408670: Fwd | CGCTCTTCCGATCTCTGTGTCTCCCCCTTTTTAGTTG | 47 |
| chr21: 30408670: Rev | CGCTCTTCCGATCTGACTTTACCTGGCTTTGGAGTTG | 48 |
| chr21: 30464866: Fwd | CGCTCTTCCGATCTCTGGTCAGCAAGTTGGCTACTGG | 49 |
| chr21: 30464866: Rev | CGCTCTTCCGATCTGACAGCCTTAGGCTCCCATGGTG | 50 |
| chr21: 31709691: Fwd | CGCTCTTCCGATCTCTGTGCTGAGTGCTTTCTGATTG | 51 |
| chr21: 31709691: Rev | CGCTCTTCCGATCTGACTCACAGACGATAGCTGTGTG | 52 |
| chr21: 31812772: Fwd | CGCTCTTCCGATCTCTGCTTCTCCTCCTGCTGTTTTG | 53 |
| chr21: 31812772: Rev | CGCTCTTCCGATCTGACCCAAAGTGCAGGATGTCTGG | 54 |
| chr21: 32253513: Fwd | CGCTCTTCCGATCTCTGGAGACTCCTCCCACTGGTTG | 55 |
| chr21: 32253513: Rev | CGCTCTTCCGATCTGACAACCCTTGCCAGGTGACTTG | 56 |
| chr21: 32638549: Fwd | CGCTCTTCCGATCTCTGTCTTCAGCAGCAGCTGGTGG | 57 |
| chr21: 32638549: Rev | CGCTCTTCCGATCTGACCCAATTCCTTGGGTGACTTG | 58 |
| chr21: 33694120: Fwd | CGCTCTTCCGATCTCTGGCGGAACCCATGTACCTGTG | 59 |
| chr21: 33694120: Rev | CGCTCTTCCGATCTGACAAACAGAGCAGAAGTGGGTG | 60 |

In addition, regarding the primer consisting of a base sequence of SEQ ID No: 41 and the primer consisting of a base sequence of SEQ ID No: 42, local alignment performed under the condition of inclusion of the 3' terminal according to the method for designing primer sets in the present invention, a local alignment score, global alignment performed on three bases of the 3' terminal of the primers according to the method for designing primer sets in the present invention, and a global alignment score are shown in Fig. 4.

As shown in Fig. 4, the base sequence of SEQ ID No: 41 and the base sequence of SEQ ID No: 42 had a local alignment score of "-3" and global alignment score of "-3", both of which were less than the set threshold value.

[Table 16]

| Primer set for X chromosome | | |
|---|---|---|
| Primer name | Base sequence (5' → 3') | SEQ ID No |
| chrX: 2779570: Fwd | CGCTCTTCCGATCTCTGAGACTCACTCCACGTGTGTG | 61 |
| chrX: 2779570: Rev | CGCTCTTCCGATCTGACAGAACCCAGTGGTGAATTTG | 62 |
| chrX: 2951434: Fwd | CGCTCTTCCGATCTCTGCTTCCCCTTCTGTGGGTGTG | 63 |
| chrX: 2951434: Rev | CGCTCTTCCGATCTGACAGGATAAAACAATGGGTTGG | 64 |
| chrX: 3241050: Fwd | CGCTCTTCCGATCTCTGCTGTTGCCGTCTCTTCATGG | 65 |
| chrX: 3241050: Rev | CGCTCTTCCGATCTGACACCTCTGGAGGAAGTTGTTG | 66 |
| chrX: 6995417: Fwd | CGCTCTTCCGATCTCTGGAACTCCTTGTGGCGGCTTG | 67 |
| chrX: 6995417: Rev | CGCTCTTCCGATCTGACCCTGCAAGAAGGTCTTATGG | 68 |

(continued)

| Primer set for X chromosome | | |
|---|---|---|
| Primer name | Base sequence (5' → 3') | SEQ ID No |
| chrX: 14027177: Fwd<br>chrX: 14027177: Rev | CGCTCTTCCGATCTCTGCTCCATGGCTTGGATCTTGG<br>CGCTCTTCCGATCTGACAGCGCCTGGACAGCTATGTG | 69<br>70 |
| chrX: 16168467: Fwd<br>chrX: 16168467: Rev | CGCTCTTCCGATCTCTGTACGCCAGGTGTCTCGCTTG<br>CGCTCTTCCGATCTGACTCCAGATAAAGGCGGCTTTG | 71<br>72 |
| chrX: 16627756: Fwd<br>chrX: 16627756: Rev | CGCTCTTCCGATCTCTGGACAGTTTGCAACCCTGTTG<br>CGCTCTTCCGATCTGACTCTGCTTTAATCGCATCGTG | 73<br>74 |
| chrX: 17746244: Fwd<br>chrX: 17746244: Rev | CGCTCTTCCGATCTCTGTGCAGGTCTGGAGGAAGTTG<br>CGCTCTTCCGATCTGACTACCAGGCACTTTGTCATGG | 75<br>76 |
| chrX: 19375782: Fwd<br>chrX: 19375782: Rev | CGCTCTTCCGATCTCTGTAATAAAGGGCCTGCGTTTG<br>CGCTCTTCCGATCTGACCACTCATACTGTGTCCGTGG | 77<br>78 |
| chrX: 22291606: Fwd<br>chrX: 22291606: Rev | CGCTCTTCCGATCTCTGAGTTACCAGCGCTTCGCTTG<br>CGCTCTTCCGATCTGACATGTTGCACAGACGGTAGTG | 79<br>80 |

In addition, regarding the primer consisting of a base sequence of SEQ ID No: 61 and the primer consisting of a base sequence of SEQ ID No: 62, local alignment performed under the condition of inclusion of the 3' terminal according to the method for designing primer sets in the present invention, a local alignment score, global alignment performed on three bases of the 3' terminal of the primers according to the method for designing primer sets in the present invention, and a global alignment score are shown in Fig. 5.

As shown in Fig. 5, the base sequence of SEQ ID No: 61 and the base sequence of SEQ ID No: 62 had a local alignment score of "-4" and global alignment score of "-3", both of which were less than the set threshold value.

[Table 17]

| Primer set for Y chromosome | | |
|---|---|---|
| Primer name | Base sequence (5' → 3') | SEQ ID No |
| chrY: 2710802: Fwd<br>chrY: 2710802: Rev | CGCTCTTCCGATCTCTGAACAAGGGCAAGAAGTTGTG<br>CGCTCTTCCGATCTGACCACCATCAATGTGGAAATTG | 81<br>82 |
| chrY: 6740540: Fwd<br>chrY: 6740540: Rev | CGCTCTTCCGATCTCTGCTTCAGCACAGATGTTTTGG<br>CGCTCTTCCGATCTGACTTTTGTTTGCCTGCCTTGTG | 83<br>84 |
| chrY: 7262370: Fwd<br>chrY: 7262370: Rev | CGCTCTTCCGATCTCTGTGTCATCAATAGTTGGCTGG<br>CGCTCTTCCGATCTGACAGTTCCCTTTTGTAGGGTGG | 85<br>86 |
| chrY: 8624945: Fwd<br>chrY: 8624945: Rev | CGCTCTTCCGATCTCTGTTGCTGTGTGAAGTCCTTGG<br>CGCTCTTCCGATCTGACAAGCGGACAGCTGTGTCTGG | 87<br>88 |
| chrY: 8632095: Fwd<br>chrY: 8632095: Rev | CGCTCTTCCGATCTCTGCCTGGGAGCTCGTGAGTTTG<br>CGCTCTTCCGATCTGACTCACGTCTGCCTAGATTTTG | 89<br>90 |
| chrY: 14655058: Fwd<br>chrY: 14655058: Rev | CGCTCTTCCGATCTCTGCAGAGTATCAGGCCTTCTGG<br>CGCTCTTCCGATCTGACGGCTTACCAGCTTGTAGTGG | 91<br>92 |
| chrY: 14756402: Fwd<br>chrY: 14756402: Rev | CGCTCTTCCGATCTCTGCCAACCATCACGAAAATTGG<br>CGCTCTTCCGATCTGACTCGTCTCGTACTGGAGATTG | 93<br>94 |
| chrY: 14944834: Fwd<br>chrY: 14944834: Rev | CGCTCTTCCGATCTCTGTGATACTCCAATTGTGGTGG<br>CGCTCTTCCGATCTGACCTGTGTTTTTCTTTGCGGTG | 95<br>96 |
| chrY: 15872674: Fwd<br>chrY: 15872674: Rev | CGCTCTTCCGATCTCTGCCCCAGTGGACAGAGTTTTG<br>CGCTCTTCCGATCTGACGGAGCCAATGCTGTGATGTG | 97<br>98 |

(continued)

| Primer set for Y chromosome | | |
|---|---|---|
| Primer name | Base sequence (5' → 3') | SEQ ID No |
| chrY: 22902924: Fwd | CGCTCTTCCGATCTCTGTGACATTGAAGGTAGCGTTG | 99 |
| chrY: 22902924: Rev | CGCTCTTCCGATCTGACGAGAAATCGGAGTTCATTGG | 100 |

In addition, regarding the primer consisting of a base sequence of SEQ ID No: 81 and the primer consisting of a base sequence of SEQ ID No: 82, local alignment performed under the condition of inclusion of the 3' terminal according to the method for designing primer sets in the present invention, a local alignment score, global alignment performed on three bases of the 3' terminal of the primers according to the method for designing primer sets in the present invention, and a global alignment score are shown in Fig. 6.

As shown in Fig. 6, the base sequence of SEQ ID No: 81 and the base sequence of SEQ ID No: 82 had a local alignment score of "-4" and global alignment score of "-3", both of which were less than the set threshold value.

<Singleplex PCR> (not according to the invention, but described for illustrative purposes)

It was confirmed that primer sets employed by performing singleplex PCR through the following procedure could amplify target loci.

2 μL of genomic DNA (0.5 ng/μL) prepared from a large number of cells (including cells having a Y chromosome), 2 μL of a primer mix, 12.5 μL of a multiplex PCR mix 2 (manufactured by TAKARA BIO INC.), 0.125 μL of a multiplex PCR mix 1 (manufactured by TAKARA BIO INC.), and a proper amount of water were mixed with each other to prepare 25 μL of a final amount of a reaction solution.

The above-described primer mix is a mix obtained by mixing primers of primer sets such that the final concentration of the primers becomes 50 nM. The above-described multiplex PCR mix 1 and the above-described multiplex PCR mix 2 are reagents contained in MULTIPLEX PCR ASSAY KIT (manufactured by TAKARA BIO INC.).

After performing initial thermal denaturation for 60 seconds at 94°C using each of the prepared reaction solutions, a thermal cycle of thermal denaturation performed for 30 seconds at 94°C, annealing performed for 90 seconds at 60°C, and an elongation reaction performed for 30 seconds at 72°C was repeated 30 cycles to perform singleplex PCR.

A part of the reaction solution on which the singleplex PCR was performed was subjected to agarose gel electrophoresis to check whether or not amplification has been performed.

<Multiplex PCR>

(Amplification of Target Loci)

2 μL of extracted genomic DNA (0.5 ng/μL), 2 μL of a primer mix, 12.5 μL of a multiplex PCR mix 2 (manufactured by TAKARA BIO INC.), 0.125 μL of a multiplex PCR mix 1 (manufactured by TAKARA BIO INC.), and a proper amount of water were mixed with each other to prepare 25 μL of a final amount of a reaction solution.

The above-described primer mix is a mix obtained by mixing a primer set for amplifying loci at 181 positions on chromosome 13, a primer set for amplifying loci at 178 positions on chromosome 18, a primer set for amplifying loci at 188 positions on chromosome 21, a primer set for amplifying loci at 51 positions on an X chromosome, and a primer set for amplifying loci at 49 positions on a Y chromosome with each other such that the final concentration of the primers becomes 50 nM. In addition, the above-described multiplex PCR mix 1 and the above-described multiplex PCR mix 2 are reagents contained in MULTIPLEX PCR ASSAY KIT (manufactured by TAKARA BIO INC.).

After performing initial thermal denaturation for 60 seconds at 94°C using each of the prepared reaction solution, a thermal cycle of thermal denaturation performed for 30 seconds at 94°C, annealing performed for 90 seconds at 60°C, and an elongation reaction performed for 30 seconds at 72°C was repeated 35 cycles to perform multiplex PCR.

<DNA Sequencing>

(Purification of PCR Amplification Product)

PCR amplification products obtained through multiplex PCR were purified using a spin column (QIAquick PCR Purification Kit manufactured by QIAGEN). In addition, the PCR amplification products may be purified using magnetic beads (for example, AMPure manufactured by Beckman Coulter Inc.).

(Addition of Index Sequence and Sequence for Bonding Flow Cell)

Next, an index sequence for identifying a sample, and P5 and P7 sequences for bonding a flow cell were added to both terminals of the multiplex PCR amplification products in order to perform a sequencing reaction using Miseq (manufactured by Illumina, Inc.). 1.25 μM of each D501-F (SEQ ID No: 101), D701-R (SEQ ID No: 102), D702-R (SEQ ID No: 103), D703-R (SEQ ID No: 104), D704-R (SEQ ID No: 105), D705-R (SEQ ID No: 106), and D706-R (SEQ ID No: 107) which were shown in Table 18 as primers, PCR amplification products obtained through multiplex PCR, a multiplex PCR mix 1, a multiplex PCR mix 2, and water were mixed with each other to prepare a reaction solution.

After performing initial thermal denaturation for 3 minutes at 94°C using each of the prepared reaction solution, a thermal cycle of thermal denaturation performed for 30 seconds at 94°C, annealing performed for 60 seconds at 50°C, and an elongation reaction performed for 30 seconds at 72°C was performed 5 cycles and a thermal cycle of thermal denaturation performed for 45 seconds at 94°C, annealing performed for 60 seconds at 55°C, and an elongation reaction performed for 30 seconds at 72°C was further performed 11 cycles. The above-described multiplex PCR mix 1 and the above-described multiplex PCR mix 2 are reagents contained in MULTIPLEX PCR ASSAY KIT (manufactured by TAKARA BIO INC.).

[Table 18]

| Primer name | Base sequence (5' → 3') | SEQ ID No |
|---|---|---|
| D501-F | AATGATACGGCGACCACCGAGATCTACACTATAGCCTTCTTTCCCTACACGACGCTCTTCCGATCTCTG | 101 |
| D701-R | CAAGCAGAAGACGGCATACGAGATCGAGTAATGTGACTGGAGTTCAGACGTGTGCTCTTCCGATCTGAC | 102 |
| D702-R | CAAGCAGAAGACGGCATACGAGATTCTCCGGAGTGACTGGACTTCAGACGTGTGCTCTTCCGATCTGAC | 103 |
| D703-R | GAAGCAGAAGAGGGCATAGGAGATAATGAGCGGTGACTGGAGTTCAGACGTGTGCTCTTCCGATCTGAC | 104 |
| D704-R | CAAGCAGAAGACGGCATACGAGATGGAATCTCGTGACTGGAGTTCAGACGTGTGCTCTTCCGATCTGAC | 105 |
| D705-R | CAAGCAGAAGACGGCATACGAGATTTCTGAATGTGACTGGAGTTCAGACGTGTGCTCTTCCGATCTGAC | 106 |
| D706-R | CAAGCAGAAGACGGCATACGAGATACGAATTCGTGACTGGAGTTCAGACGTGTGCTCTTCCGATCTGAC | 107 |

The PCR amplification products obtained through multiplex PCR were purified using DNA purification reagent kit AMPure XP (manufactured by Beckman Coulter Inc.) and the concentrations thereof were measured using Agilent 2100 BIOANALYZER (manufactured by Agilent Technologies).

Quantitative determination was performed as more accurate quantitative determination of amplification products using KAPA LIBRARY QUANTIFICATION KIT (manufactured by NIPPON Genetics Co, Ltd.).

(Measurement of Number of Times of Sequence Reading (Coverage, Sequence Depth))

The coverage (sequence depth) for each target locus of chromosome 13, chromosome 18, and chromosome 21 of nucleated red blood cells which were identified as being derived from a fetus was measured by performing sequencing of amplification products using a next generation sequencer MiSeq (registered trademark manufactured by Illumina, Inc.). The amount of amplification products (number of times of sequence reading) of target loci of chromosome 21 of nucleated cells which were identified as being derived from a mother were separately measured by performing sequencing of the amplification products using Miseq.

The coverage (sequence depth) was calculated for each locus and the variation in coverage was evaluated using the coefficient of variation (CV). As a result, the coefficient of variation was about 6.5% which indicates small variation in coverage.

[Comparative Example 1]

Although the point that chromosomes of interest are chromosome 13, chromosome 18, and chromosome 21 is not different from that of Example 1, the number of loci on chromosome 13 was set to 75, the number of loci on chromosome 18 was set to 77, the number of loci on chromosome 21 was set to 76, the number of loci on an X chromosome was set to 34, and the number of loci on a Y chromosome was set to 20, so that the total number of loci became 282.

The coverage was calculated for each locus while setting the other conditions to be the same as those in Example 1, and the variation in coverage was evaluated using the coefficient of variation. As a result, the coefficient of variation was about 28.2% which indicates large variation in coverage.

[Comparative Example 2]

Although the point that chromosomes of interest are chromosome 13, chromosome 18, and chromosome 21 is not different from that of Example 1, the number of loci on chromosome 13 was set to 52, the number of loci on chromosome 18 was set to 49, the number of loci on chromosome 21 was set to 46, the number of loci on an X chromosome was set to 34, and the number of loci on a Y chromosome was set to 20, so that the total number of loci became 201.

The coverage was calculated for each locus while setting the other conditions to be the same as those in Example 1, and the variation in coverage was evaluated using the coefficient of variation. As a result, the coefficient of variation was about 32.7% which indicates large variation in coverage.

[Comparative Example 3]

Although the point that chromosomes of interest are chromosome 13, chromosome 18, and chromosome 21 is not different from that of Example 1, the number of loci on chromosome 13 was set to 20, the number of loci on chromosome 18 was set to 20, the number of loci on chromosome 21 was set to 20, the number of loci on an X chromosome was set to 20, and the number of loci on a Y chromosome was set to 20, so that the total number of loci became 100.

The coverage was calculated for each locus while setting the other conditions to be the same as those in Example 1, and the variation in coverage was evaluated using the coefficient of variation. As a result, the coefficient of variation was about 52.9% which indicates large variation in coverage.

[Comparative Example 4]

Although the point that chromosomes of interest are chromosome 13, chromosome 18, and chromosome 21 is not different from that of Example 1, the number of loci on chromosome 13 was set to 9, the number of loci on chromosome 18 was set to 9, the number of loci on chromosome 21 was set to 8, and the number of loci on an X chromosome was set to 9, so that the total number of loci became 35.

The coverage was calculated for each locus while setting the other conditions to be the same as those in Example 1, and the variation in coverage was evaluated using the coefficient of variation. As a result, the coefficient of variation was about 143.1% which indicates large variation in coverage.

Fig. 7 shows a graph in which the (total) number of loci is plotted on the lateral axis and the coefficient of variation of the coverage is plotted on the longitudinal axis. The plots correspond to Example 1 and Comparative Examples 1 to 4, and the curve is an approximate curve obtained from the plots.

In a case where the number of loci on chromosomes of interest is set to be greater than or equal to 80, the coefficient of variation of the coverage becomes sufficiently small and the variation of coverage for each locus is reduced. Therefore, it is considered that it is possible to accurately perform quantitative determination of the number of chromosomes from a small amount of DNA of a single cell, a small number of cells, or the like.

[Sequence List]

International Application W-5931PCT Method for Determining Number of Chromosomes JP17004390 20170207----00260357151700267697 Normal 20170207093843201701120942231730_P1AP101_W-_18.app Based on International Patent Cooperation Treaty

## Claims

1. A chromosome number determination method of chromosomes of interest, comprising:

   a step of performing multiplex PCR for simultaneously amplifying a plurality of loci on the chromosomes using genomic DNA extracted from a single cell or a small number of cells as templates,
   wherein the number of loci on the chromosomes of interest is greater than or equal to 80 per chromosome,
   wherein a plurality of primer sets used in the multiplex PCR are designed through a method for designing primer sets used in the polymerase chain reaction, the method for designing primer sets including
   a target locus selection step of selecting a target locus for designing primer sets used in the multiplex PCR from the plurality of loci,
   a primer candidate base sequence generation step of generating at least one base sequence of a primer candidate for amplifying the target locus regarding each of a forward-side primer and a reverse-side primer based on a base sequence in a vicinity region of the target locus on the chromosomes,
   a local alignment step of obtaining a local alignment score by performing pairwise local alignment on the base sequence of the primer candidate for amplifying the target locus under a condition that a partial sequence to be subjected to comparison includes the 3' terminal of the base sequence of the primer candidate for amplifying the target locus,
   a first stage selection step of performing first stage selection of the base sequence of the primer candidate for amplifying the target locus based on the local alignment score obtained in the local alignment step,
   a global alignment step of obtaining a global alignment score by performing pairwise global alignment on a base sequence which has a sequence length of three bases and includes the 3' terminal of the base sequence of the primer candidate for amplifying the target locus,
   a second stage selection step of performing second stage selection of the base sequence of the primer candidate for amplifying the target locus based on the global alignment score obtained in the global alignment step, and
   a primer employment step of employing the base sequence of the primer candidate which has been selected in both of the first stage selection step and the second stage selection step as the base sequence of the primer for amplifying the target locus, and
   wherein both steps of the local alignment step and the first stage selection step are performed before or after both steps of the global alignment step and the second stage selection step, or performed in parallel with both steps of the global alignment step and the second stage selection step.

2. The chromosome number determination method according to claim 1,
   wherein the number of loci on the chromosomes of interest is 80 to 1,000 per chromosome.

3. The chromosome number determination method according to claim 1 or 2,
   wherein the number of loci on the chromosomes of interest is 100 to 1,000 per chromosome.

4. The chromosome number determination method according to any one of claims 1 to 3,
   wherein the number of loci on the chromosomes of interest is 100 to 500 per chromosome.

5. The chromosome number determination method according to any one of claims 1 to 4,

wherein the chromosomes of interest contain at least one selected from the group consisting of chromosome 13, chromosome 18, and chromosome 21.

**6.** The chromosome number determination method according to any one of claims 1 to 5, wherein the steps from the target locus selection step to the primer employment step are repeated until the primer sets used in the multiplex PCR are employed for all of the plurality of loci.

**7.** The chromosome number determination method according to any one of claims 1 to 6, wherein one or more loci are selected in the target locus selection step.

**8.** The chromosome number determination method according to any one of claims 1 to 5,

wherein primer sets used in the multiplex PCR are designed through a method for designing primer sets used in the polymerase chain reaction, the method for designing primer sets including
a first target locus selection step of selecting a first target locus for designing primer sets used in the multiplex PCR from the plurality of loci,
a first primer candidate base sequence generation step of generating at least one base sequence of a primer candidate for amplifying the first target locus regarding each of a forward-side primer and a reverse-side primer based on a base sequence in a vicinity region of the first target locus on the chromosomes,
a first local alignment step of obtaining a local alignment score by performing pairwise local alignment on the base sequence of the primer candidate for amplifying the first target locus under a condition that a partial sequence to be subjected to comparison includes the 3' terminal of the base sequence of the primer candidate for amplifying the first target locus,
a first step of first stage selection of performing first stage selection of the base sequence of the primer candidate for amplifying the first target locus based on the local alignment score obtained in the first local alignment step,
a first global alignment step of obtaining a global alignment score by performing pairwise global alignment on a base sequence which has a sequence length of three bases and includes the 3' terminal of the base sequence of the primer candidate for amplifying the first target locus,
a first step of second stage selection of performing second stage selection of the base sequence of the primer candidate for amplifying the first target locus based on the global alignment score obtained in the first global alignment step,
a first primer employment step of employing the base sequence of the primer candidate which has been selected in both of the first step of first stage selection and the first step of second stage selection as a base sequence of a primer for amplifying the first target locus,
a second target locus selection step of selecting a second target locus, which is different from the already selected target locus and in which primer sets used in the multiplex PCR are designed, from the plurality of loci,
a second primer candidate base sequence generation step of generating at least one base sequence of a primer candidate for amplifying the second target locus regarding each of a forward-side primer and a reverse-side primer based on a base sequence in a vicinity region of the second target locus on the chromosomes,
a second local alignment step of obtaining a local alignment score by performing pairwise local alignment on the base sequence of the primer candidate for amplifying the second target locus and the base sequence of the primer which has already been employed, under a condition that partial sequences to be subjected to comparison include the 3' terminal of the base sequence of the primer candidate for amplifying the second target locus and the 3' terminal of the base sequence of the primer which has already been employed,
a second step of first stage selection of performing first stage selection of the base sequence of the primer candidate for amplifying the second target locus based on the local alignment score obtained in the second local alignment step,
a second global alignment step of obtaining a global alignment score by performing pairwise global alignment on base sequences which have a sequence length of three bases and include the 3' terminal of the base sequence of the primer candidate for amplifying the second target locus and the 3' terminal of the base sequence of the primer which has already been employed,
a second step of second stage selection of performing second stage selection of the base sequence of the primer candidate for amplifying the second target locus based on the global alignment score obtained in the second global alignment step, and
a second primer employment step of employing the base sequence of the primer candidate which has been selected in both of the second step of first stage selection and the second step of second stage selection as a base sequence of a primer for amplifying the second target locus,
wherein both steps of the first local alignment step and the first step of first stage selection are performed before or after both steps of the first global alignment step and the first step of second stage selection, or performed in parallel with both steps of the first global alignment step and the first step of second stage selection,
wherein both steps of the second local alignment step and the second step of first stage selection are performed before or after both steps of the second global alignment step and the second step of second stage selection, or performed in parallel with both steps of the second global alignment step and the second step of second stage selection, and
wherein, in a case where the number of the plurality of loci is three or more, the steps from the second target locus selection step to the second primer employment step are repeated until the primer sets used in the multiplex PCR are employed for all of the plurality of loci.

**Patentansprüche**

**1.** Chromosomenanzahl-Bestimmungsverfahren von Chromosomen von Interesse, aufweisend:

einen Multiplex-PCR-Durchführungsschritt zum gleichzeitig Amplifizieren einer Mehrzahl von Orten auf den Chromosomen unter Verwendung von genomischer DNA, die aus einer einzigen Zelle oder einer kleinen Anzahl von Zellen extrahiert wurde, als Matrizen,
wobei die Anzahl an Orten auf den Chromosomen von Interesse größer als oder gleich 80 pro Chromosom ist,
wobei eine Mehrzahl von Primersätzen, die in der Multiplex-PCR verwendet werden, entworfen werden durch ein Verfahren zum Entwerfen von in der Polymerase-Kettenreaktion verwendeten Primersätzen, wobei das Verfahren zum Entwerfen von Primersätzen umfasst
einen Zielort-Auswahlschritt des Auswählens eines Zielorts zum Entwerfen von Primersätzen, die in der Multiplex-PCR verwendet werden, aus der Mehrzahl von Orten,
einen Primerkandidaten-Basensequenz-Erzeugungsschritt des Erzeugens mindestens einer Basensequenz eines Primerkandidaten zum Amplifizieren des Zielorts hinsichtlich sowohl eines Vorwärtsprimers als auch eines Rückwärtsprimers auf der Basis einer Basensequenz in einer Nachbarregion des Zielorts auf den Chromosomen,
einen lokalen Alignmentschritt des Erhaltens eines lokalen Alignment-Scores durch Ausführen eines paarweisen lokalen Alignments an der Basensequenz des Primerkandidaten zum Amplifizieren des Zielorts unter einer Bedingung, dass eine Teilsequenz, die einem Vergleich unterzogen werden soll, das 3'-Ende der Basensequenz des Primerkandidaten zum Amplifizieren des Zielorts enthält,
einen Erstphasen-Auswahlschritt des Durchführens einer Erstphasen-Auswahl der Basensequenz des Primerkandidaten zum Amplifizieren des Zielorts auf der Basis des lokalen Alignment-Scores, der in dem lokalen Alignmentschritt erhalten wurde,
einen globalen Alignmentschritt des Erhaltens eines globalen Alignment-Scores durch Durchführen eines paarweisen globalen Alignments an einer Basensequenz, die eine Sequenzlänge von drei Basen hat und das 3'-Ende der Basensequenz des Primerkandidaten zum Amplifizieren des Zielorts enthält,
einen Zweitphasen-Auswahlschritt des Durchführens einer Zweitphasen-Auswahl der Basensequenz des Primerkandidaten zum Amplifizieren des Zielorts auf der Basis des globalen Alignment-Scores, der in dem globalen Alignmentschritt erhalten wurde, und
einen Primeranwendungsschritt des Anwendens der Basensequenz des Primerkandidaten, die sowohl in dem Erstphasen-Auswahlschritt als auch in dem Zweitphasen-Auswahischritt als die Basensequenz des Primers zum Amplifizieren des Zielorts ausgewählt wurde, und
wobei sowohl der lokale Alignmentschritt als auch der Erstphasen-Auswahlschritt vor oder nach sowohl dem globalen Alignmentschritt als auch dem Zweitphasen-Auswahischritt durchgeführt werden oder parallel mit sowohl dem globalen Alignmentschritt als auch dem Zweitphasen-Auswahlschritt durchgeführt werden.

**2.** Chromosomenanzahl-Bestimmungsverfahren nach Anspruch 1,
wobei die Anzahl an Orten auf den Chromosomen von Interesse 80 bis 1.000 pro Chromosom beträgt.

**3.** Chromosomenanzahl-Bestimmungsverfahren nach Anspruch 1 oder 2,
wobei die Anzahl an Orten auf den Chromosomen von Interesse 100 bis 1.000 pro Chromosom beträgt.

**4.** Chromosomenanzahl-Bestimmungsverfahren nach einem der Ansprüche 1 bis 3,
wobei die Anzahl an Orten auf den Chromosomen von Interesse 100 bis 500 pro Chromosom beträgt.

**5.** Chromosomenanzahl-Bestimmungsverfahren nach einem der Ansprüche 1 bis 4,

wobei die Chromosomen von Interesse mindestens ein Chromosom enthalten, das ausgewählt wird aus der Gruppe, die aus Chromosom 13, Chromosom 18 und Chromosom 21 besteht.

**6.** Chromosomenanzahl-Bestimmungsverfahren nach einem der Ansprüche 1 bis 5,
wobei die Schritte von dem Zielort-Auswahlschritt bis zu dem Primeranwendungsschritt wiederholt werden bis die Primersätze, die in der Multiplex-PCR verwendet werden, für alle der Mehrzahl von Orten verwendet wurden.

**7.** Chromosomenanzahl-Bestimmungsverfahren nach einem der Ansprüche 1 bis 6,
wobei in dem Zielort-Auswahlschritt ein oder mehrere Orte ausgewählt werden.

**8.** Chromosomenanzahl-Bestimmungsverfahren nach einem der Ansprüche 1 bis 5,

wobei Primersätze, die in der Multiplex-PCR verwendet werden, durch ein Verfahren zum Entwerfen von in der Polymerase-Kettenreaktion verwendeten Primersätzen entworfen werden, wobei das Verfahren zum Entwerfen von Primersätzen umfasst
einen ersten Zielort-Auswahlschritt des Auswählens eines ersten Zielorts zum Entwerfen von in der Multiplex-PCR verwendeten Primersätzen aus der Mehrzahl von Orten,
einen ersten Primerkandidaten-Basensequenz-Erzeugungsschritt des Erzeugens mindestens einer Basensequenz eines Primerkandidaten zum Amplifizieren des ersten Zielorts hinsichtlich sowohl eines Vorwärtsprimers als auch eines Rückwärtsprimers auf der Basis einer Basensequenz in einer Nachbarregion des ersten Zielorts auf den Chromosomen,
einen ersten lokalen Alignmentschritt des Erhaltens eines lokalen Alignment-Scores durch Durchführen eines paarweisen lokalen Alignments an der Basensequenz des Primerkandidaten zum Amplifizieren des ersten Zielorts unter einer Bedingung, dass eine Teilsequenz, die einem Vergleich unterzogen werden soll, das 3'-Ende der Basensequenz des Primerkandidaten zum Amplifizieren des ersten Zielorts enthält,
einen ersten Erstphasen-Auswahlschritt des Durchführens einer Erstphasen-Auswahl der Basensequenz des Primerkandidaten zum Amplifizieren des ersten Zielorts auf der Basis des lokalen Alignment-Scores, der in dem ersten lokalen Alignmentschritt erhalten wurde,
einen ersten globalen Alignementschritt des Erhaltens eines globalen Alignment-Scores durch Durchführen eines paarweisen globalen Alignments an einer Basensequenz, die eine Sequenzlänge von drei Basen hat und das 3'-Ende der Basensequenz des Primerkandidaten zum Amplifizieren des ersten Zielorts enthält,
einen ersten Zweitphasen-Auswahlschritt des Durchführens einer Zweitphasen-Auswahl der Basensequenz des Primerkandidaten zum Amplifizieren des ersten Zielorts auf der Basis des globalen Alignment-Scores, der in dem ersten globalen Alignmentschritt erhalten wurde,
eine ersten Primeranwendungsschritt des Anwendens der Basensequenz des Primerkandidaten, die sowohl in dem ersten Erstphasen-Auswahlschritt als auch in dem ersten Zweitphasen-Auswahlschritt als eine Basensequenz eines Primers zum Amplifizieren des ersten Zielorts ausgewählt wurde,
einen zweiten Zielort-Auswahlschritt des Auswählens eines zweiten Zielorts, der von dem bereits ausgewählten Zielort verschieden ist und in dem in der Multiplex-PCR verwendete Primersätze entworfen werden, aus der Mehrzahl von Orten,
einen zweiten Primerkandidaten-Basensequenz-Erzeugungsschritt des Erzeugens mindestens einer Basensequenz eines Primerkandidaten zum Amplifizieren des zweiten Zielorts hinsichtlich sowohl einer Vorwärtsprimers als auch eines Rückwärtsprimers auf der Basis einer Basensequenz in einer Nachbarregion des zweiten Zielorts auf den Chromosomen,
einen zweiten lokalen Alignmentschritt des Erhaltens eines lokalen Alignment-Scores durch Durchführen eines paarweisen lokalen Alignments an der Basensequenz des Primerkandidaten zum Amplifizieren des zweiten Zielorts und der Basensequenz des Primers, der bereits angewendet wurde, unter der Bedingung, dass zu vergleichende Teilsequenzen das 3'-Ende der Basensequenz des Primerkandidaten zum Amplifizieren des zweiten Zielorts und das 3'-Ende der Basensequenz des Primers, der bereits angewendet wurde, enthalten,
einen zweiten Erstphasen-Auswahlschritt des Durchführens einer Erstphasen-Auswahl der Basensequenz des Primerkandidaten zum Amplifizieren des zweiten Zielorts auf der Basis des lokalen Alignment-Scores, der in dem zweiten lokalen Alignmentschritt erhalten wurde,
einen zweiten globalen Alignmentschritt des Erhaltens eines globalen Alignment-Scores durch Durchführen eines paarweisen globalen Alignments an Basensequenzen, die eine Sequenzlänge von drei Basen haben und das 3'-Ende der Basensequenz des Primerkandidaten zum Amplifizieren des zweiten Zielorts und das 3'-Ende der Basensequenz des Primers, der bereits verwendet wurde, enthalten,
einen zweiten Zweitphasen-Auswahlschritt des Durchführens einer Zweitphasen-Auswahl der Basensequenz des Primerkandidaten zum Amplifizieren des zweiten Zielorts auf der Basis des globalen Alignment-Scores, der in dem zweiten globalen Alignmentschritt erhalten wurde, und
einen zweiten Primeranwendungsschritt des Anwendens der Basensequenz des Primerkandidaten, die sowohl in dem zweiten Erstphasen-Auswahlschritt als auch in dem zweiten Zweitphasen-Auswahlschritt als eine Basensequenz eines Primers zum Amplifizieren des zweiten Zielorts ausgewählt wurde,
wobei sowohl der erste lokale Alignmentschritt als auch der erste Erstphasen-Auswahlschritt vor oder nach sowohl dem ersten globalen Alignmentschritt als auch dem ersten Zweitphasen-Auswahlschritt durchgeführt werden, oder parallel mit sowohl dem ersten globalen Alignmentschritt als auch dem ersten Zweitphasen-Auswahlschritt durchgeführt werden,
wobei sowohl der zweite lokale Alignmentschritt als auch der zweite Erstphasen-Auswahlschritt vor oder nach sowohl dem zweiten globalen Alignmentschritt als auch dem zweiten Zweitphasen-Auswahlschritt durchgeführt werden, oder parallel sowohl mit zweiten globalen Alignmentschritt als auch dem zweiten Zweitphasen-auswahlschritt durchgeführt werden, und
wobei in einem Fall, in dem die Anzahl der Mehrzahl von Orten drei oder mehr beträgt, die Schritte vom zweiten Zielort-Auswahlschritt bis zum zweiten Primeranwendungsschritt wiederholt werden bis die Primersätze, die in der Multiplex-PCR verwendet werden, für alle der Mehrzahl von Orten angewendet sind.

## Revendications

**1.** Procédé de détermination de nombre de chromosomes de chromosomes d'intérêt, comprenant les étapes suivantes :

une étape pour réaliser une amplification en chaîne par polymérase (Polymerase Chain Reaction, PCR) multiplexe pour amplifier simultanément une pluralité de locus sur les chromosomes à l'aide de l'ADN génomique extrait d'une cellule unique ou d'un petit nombre de cellules comme matrices,
dans lequel le nombre de locus sur les chromosomes d'intérêt est supérieur ou égal à 80 par chromosome ;
dans lequel une pluralité de jeux d'amorces utilisés dans la PCR multiplexe sont conçus par le biais d'un procédé pour concevoir des jeux d'amorce utilisés dans la réaction en chaîne par polymérase, le procédé pour concevoir les jeux d'amorces incluant
une étape de sélection de locus cible pour sélectionner un locus cible pour concevoir des jeux d'amorces utilisés dans la PCR multiplexe parmi la pluralité de locus ;
une étape de génération de séquence de bases d'amorce candidate pour générer au moins une séquence de bases d'une amorce candidate pour amplifier le locus cible concernant chaque amorce parmi une amorce côté avant et une amorce côté arrière sur la base d'une séquence de bases dans une région voisine du locus cible sur les chromosomes ;
une étape d'alignement local pour obtenir un score d'alignement local en réalisant un alignement local par paires sur la séquence de bases de l'amorce candidate pour amplifier le locus cible à condition qu'une séquence partielle à soumettre à comparaison inclue l'extrémité 3' de la séquence de bases de l'amorce candidate pour amplifier le locus cible ;
une étape de sélection de premier stade pour réaliser une sélection de premier stade de la séquence de bases de l'amorce candidate pour amplifier le locus cible sur la base du score d'alignement local obtenu lors de l'étape d'alignement local ;
une étape d'alignement global pour obtenir un score d'alignement global en réalisant un alignement global par paires sur une séquence de bases, laquelle présente une longueur de séquence de trois bases et inclut l'extrémité 3' de la séquence de bases de l'amorce candidate pour amplifier le locus cible ;
une étape de sélection de second stade pour réaliser une sélection de second stade de la séquence de bases de l'amorce candidate pour amplifier le locus cible sur la base du score d'alignement global obtenu lors de l'étape d'alignement global, et
une étape d'emploi d'amorce pour employer la séquence de bases de l'amorce candidate, laquelle a été sélectionnée lors des deux étapes parmi l'étape de sélection de premier stade et l'étape de sélection de second stade comme séquence de bases de l'amorce pour amplifier le locus cible, et
dans lequel les deux étapes parmi l'étape d'alignement local et l'étape de sélection de premier stade sont réalisées avant ou après les deux étapes parmi l'étape d'alignement global et l'étape de sélection de second stade, ou sont réalisées parallèlement aux deux étapes parmi l'étape d'alignement global et l'étape de sélection de second stade.

**2.** Procédé de détermination de nombre de chromosomes selon la revendication 1,
dans lequel le nombre de locus sur les chromosomes d'intérêt s'étend de 80 à 1000 par chromosome.

**3.** Procédé de détermination de nombre de chromosomes selon la revendication 1 ou 2, dans lequel le nombre de locus sur les chromosomes d'intérêt s'étend de 100 à 1000 par chromosome.

**4.** Procédé de détermination de nombre de chromosomes selon l'une quelconque des revendications 1 à 3, dans lequel le nombre de locus sur les chromosomes d'intérêt s'étend de 100 à 500 par chromosome.

**5.** Procédé de détermination de nombre de chromosomes selon l'une quelconque des revendications 1 à 4, dans lequel les chromosomes d'intérêt contiennent au moins un chromosome sélectionné parmi le groupe consistant en le chromosome 13, le chromosome 18, et le chromosome 21.

**6.** Procédé de détermination de nombre de chromosomes selon l'une quelconque des revendications 1 à 5, dans lequel les étapes allant de l'étape de sélection de locus cible à l'étape d'emploi d'amorce sont répétées jusqu'à ce que les jeux d'amorces utilisés dans la PCR multiplexe soient employés pour la totalité de la pluralité de locus.

**7.** Procédé de détermination de nombre de chromosomes selon l'une quelconque des revendications 1 à 6, dans lequel un ou plusieurs locus sont sélectionnés lors de l'étape de sélection de locus cible.

**8.** Procédé de détermination de nombre de chromosomes selon l'une quelconque des revendications 1 à 5,

dans lequel des jeux d'amorces utilisés pour la PCR multiplexe sont conçus par le biais d'un procédé pour concevoir des jeux d'amorces utilisés dans la réaction en chaîne par polymérase, le procédé pour concevoir des jeux d'amorce incluant

une première étape de sélection de locus cible pour sélectionner un premier locus cible pour concevoir des jeux d'amorces utilisés dans la PCR multiplexe parmi la pluralité de locus ;

une première étape de génération de séquence de bases d'amorce candidate pour générer au moins une séquence de bases d'une amorce candidate pour amplifier le premier locus cible concernant chaque amorce parmi une amorce côté avant et une amorce côté arrière sur la base d'une séquence de bases dans une région voisine du premier locus cible sur les chromosomes ;

une première étape d'alignement local pour obtenir un score d'alignement local en réalisant un alignement local par paires sur la séquence de bases de l'amorce candidate pour amplifier le premier locus cible à condition qu'une séquence partielle à soumettre à comparaison inclue l'extrémité 3' de la séquence de bases de l'amorce candidate pour amplifier le premier locus cible ;

une première étape de sélection de premier stade pour réaliser une sélection de premier stade de la séquence de bases de l'amorce candidate pour amplifier le premier locus cible sur la base du score d'alignement local obtenu lors de la première étape d'alignement local ;

une première étape d'alignement global pour obtenir un score d'alignement global en réalisant un alignement global par paires sur une séquence de bases, laquelle présente une longueur de séquence de trois bases et inclut l'extrémité 3' de la séquence de bases de l'amorce candidate pour amplifier le premier locus cible ;

une première étape de sélection de second stade pour réaliser une sélection de second stade de la séquence de bases de l'amorce candidate pour amplifier le premier locus cible sur la base du score d'alignement global obtenu lors de la première étape d'alignement global ;

une première étape d'emploi d'amorce pour employer la séquence de bases de l'amorce candidate, laquelle a été sélectionnée lors des deux étapes parmi la première étape de sélection de premier stade et la première étape de sélection de second stade comme séquence de bases d'une amorce pour amplifier le premier locus cible ;

une seconde étape de sélection de locus cible pour sélectionner un second locus cible, lequel est différent du locus cible déjà sélectionné, et où des jeux d'amorce utilisés dans la PCR multiplexe sont conçus, parmi la pluralité de locus ;

une seconde étape de génération de séquence de bases d'amorce candidate pour générer au moins une séquence de bases d'une amorce candidate pour amplifier le second locus cible concernant chaque amorce parmi une amorce côté avant et une amorce côté arrière sur la base d'une séquence de bases dans une région voisine du second locus cible sur les chromosomes ;

une seconde étape d'alignement local pour obtenir un score d'alignement local en réalisant un alignement local par paires sur la séquence de bases de l'amorce candidate pour amplifier le second locus cible et la séquence de bases de l'amorce ayant déjà été employée, à condition qu'une séquence partielle à soumettre à comparaison inclue l'extrémité 3' de la séquence de bases de l'amorce candidate pour amplifier le second locus cible et l'extrémité 3' de la séquence de bases de l'amorce candidate ayant déjà été employée ;

une seconde étape de sélection de premier stade pour réaliser une sélection de premier stade de la séquence de bases de l'amorce candidate pour amplifier le second locus cible sur la base du score d'alignement local obtenu lors de la seconde étape d'alignement local ;
une seconde étape d'alignement global pour obtenir un score d'alignement global en réalisant un alignement global par paires sur des séquences de bases, lesquelles présentent une longueur de séquence de trois bases et inclut l'extrémité 3' de la séquence de bases de l'amorce candidate pour amplifier le second locus cible et l'extrémité 3' de la séquence de bases de l'amorce ayant déjà été employée ;
une seconde étape de sélection de second stade pour réaliser une sélection de second stade de la séquence de bases de l'amorce candidate pour amplifier le second locus cible sur la base du score d'alignement global obtenu lors de la seconde étape d'alignement global, et
une seconde étape d'emploi d'amorce pour employer la séquence de bases de l'amorce candidate, laquelle a été sélectionnée lors des deux étapes parmi la seconde étape de sélection de premier stade et la seconde étape de sélection de second stade comme séquence de bases d'une amorce pour amplifier le second locus cible ;
dans lequel les deux étapes parmi la première étape d'alignement local et la première étape de sélection de premier stade sont réalisées avant ou après les deux étapes parmi la première étape d'alignement global et la première étape de sélection de second stade, ou sont réalisées parallèlement aux deux étapes parmi la première étape d'alignement global et la première étape de sélection de second stade ;
dans lequel les deux étapes parmi la seconde étape d'alignement local et la seconde étape de sélection de premier stade sont réalisées avant ou après les deux étapes parmi la seconde étape d'alignement global et la seconde étape de sélection de second stade, ou sont réalisées parallèlement aux deux étapes parmi la seconde étape d'alignement global et la seconde étape de sélection de second stade, et
dans lequel dans un cas où le nombre de la pluralité de locus est supérieur ou égal à trois, les étapes allant de la seconde étape de sélection de locus cible jusqu'à la seconde étape d'emploi d'amorce sont répétées jusqu'à ce que les jeux d'amorces utilisés dans la PCR multiplexe soient employés pour la totalité de la pluralité de locus.

FIG. 1

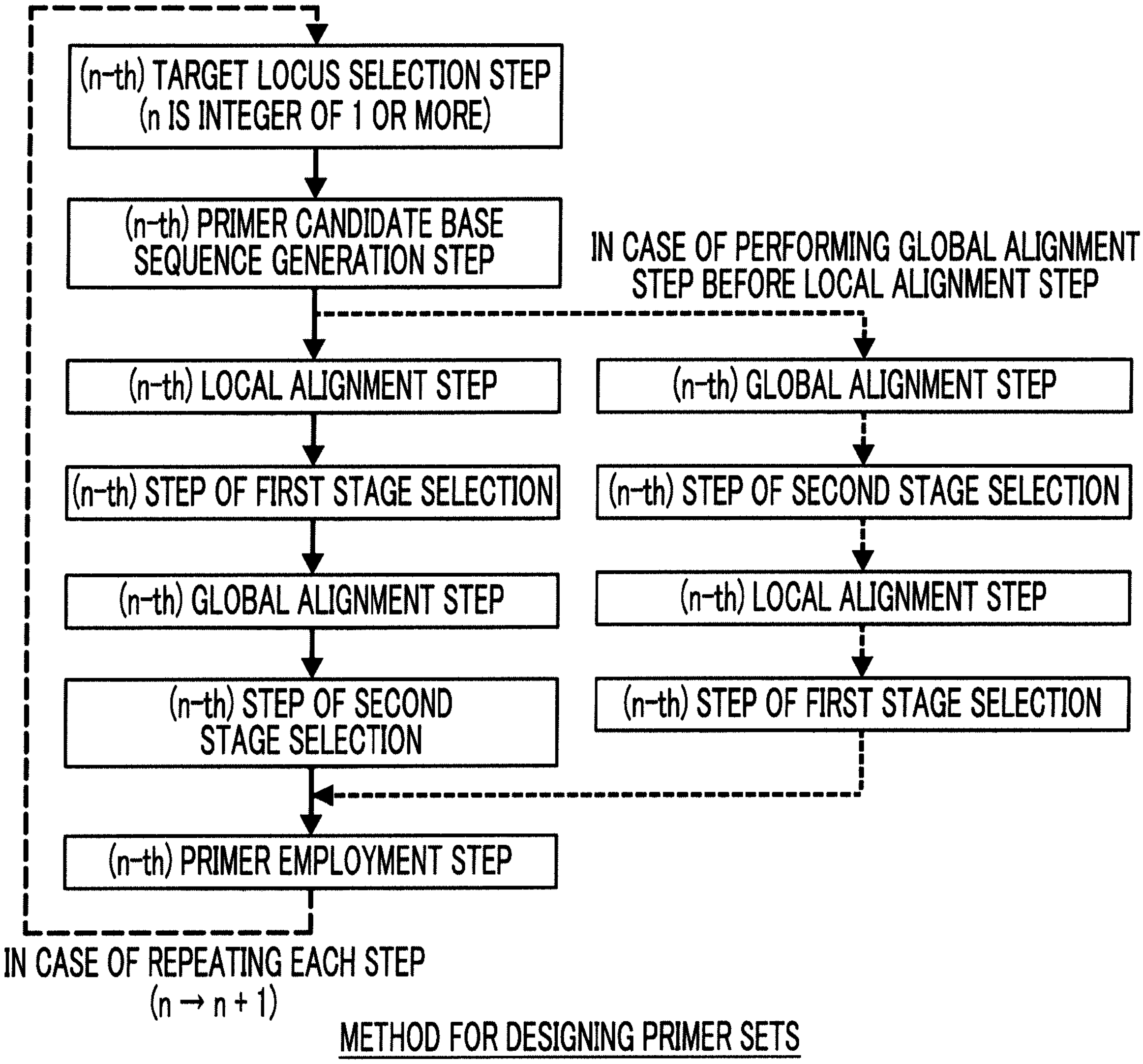


FIG. 2

```
Local alignment score = -8
[SEQ ID:1]  5'~cgctcttccgatctctgCTTCGATGCGGACCTTCTGG              ~3'
                                |:::::::*|||||::|::::|
[SEQ ID:2]  3'~                 GGTATCG-GCCTACACCCTCtcagtctagccttctcgc~5'
("|"=match; ":"=mismatch; "*"=gap)

Global alignment score = -3
[SEQ ID:1]  5'~TGG~3'
               :::
[SEQ ID:2]  3'~GGT~5'
("|"=match; ":"=mismatch; "*"=gap)
```

## FIG. 3

```
Local alignment score = -7
[SEQ ID:21]  5'~cgctcttccgatctctgAGG-TTCTGCTCGTTGGCTTG                  ~3'
                                 :::*|:|:::||:||**||||
[SEQ ID:22]  3'~                 GGTTAGGCACAGGAA--GAACccagtctagccttctcgc~5'
("|"=match; ":"=mismatch; "*"=gap)

Global alignment score = -3
[SEQ ID:21]  5'~TTG~3'
                :::
[SEQ ID:22]  3'~GGT~5'
("|"=match; ":"=mismatch; "*"=gap)
```

## FIG. 4

```
Local alignment score = -3
[SEQ ID:41]  5'~cgctcttccgatctctggCAACAGTCTGGCTTTTTG                   ~3'
                                  ||||:|:::::|:|::|::
[SEQ ID:42]  3'~                  GTTGCCGTCATGGACTAGGgcagtctagcccttctcgc~5'
("|"=match; ":"=mismatch; "*"=gap)

Global alignment score = -3
[SEQ ID:41]  5'~TTG~3'
                :::
[SEQ ID:42]  3'~GTT~5'
("|"=match; ":"=mismatch; "*"=gap)
```

## FIG. 5

```
Local alignment score = -4
[SEQ ID:61]  5'~cgctcttccgatctctgaGACTCACTCCAC-GTGTGTG                ~3'
                                  :|::::|:||||*|:||:::
[SEQ ID:62]  3'~                  GTTTAAGTGGTGACCCAAGAcagtctagccttctcgc~5'
("|"=match; ":"=mismatch; "*"=gap)

Global alignment score = -3
[SEQ ID:61]  5'~GTG~3'
                :::
[SEQ ID:62]  3'~GTT~5'
("|"=match; ":"=mismatch; "*"=gap)
```

## FIG. 6

```
Local alignment score = -4
[SEQ ID:81]  5'~cgctcttccgatctctgaaCAAGGGCAAGA--AGTTGTG               ~3'
                                   |||:::|:|:|**::|:|||
[SEQ ID:82]  3'~                   GTTAAAGGTGTAACTACCACcagtctagccttctcgc~5'
("|"=match; ":"=mismatch; "*"=gap)

Global alignment score = -3
[SEQ ID:81]  5'~GTG~3'
                :::
[SEQ ID:82]  3'~GTT~5'
("|"=match; ":"=mismatch; "*"=gap)
```

FIG. 7

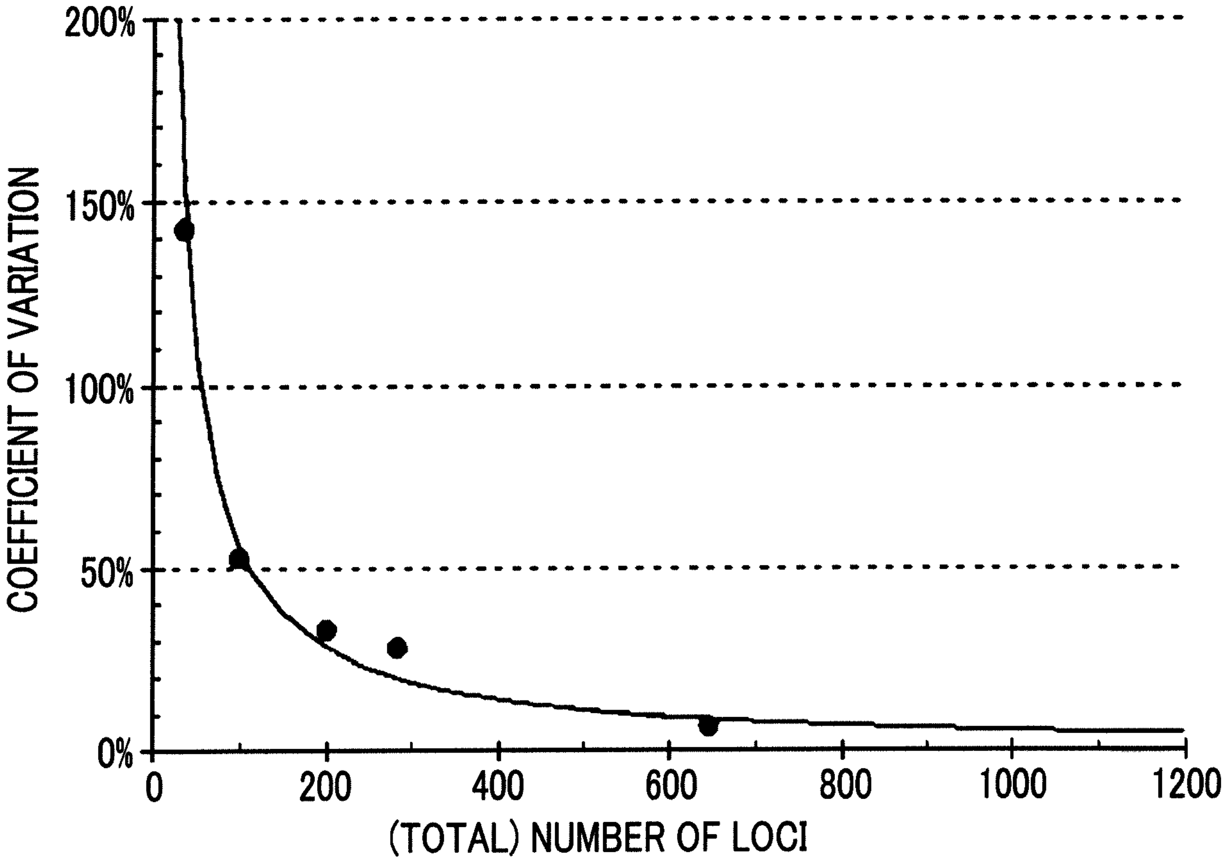
200%
150%
100%
50%
0%
COEFFICIENT OF VARIATION
0
200
400
600
800
1000
1200
(TOTAL) NUMBER OF LOCI

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014018080 A
- WO 2008004691 A
- US 2010070452 A1
- WO 2012023298 A
- JP 2016033284 A

### Non-patent literature cited in the description


- **ZIMMERMANN B et al.** P-448 One day turn around 24-chromosome preimplantation genetic diagnosis by targeted sequencing enabled by multiplex PCR and fast benchtop sequencers. *HUMAN REPRODUCTION, OXFORD JOURNALS, GB,* 01 January 2012, vol. 27 (2), 448
- **LI, H. et al.** Fast and accurate short read alignment with Burrows-Wheeler transform. *Bioinformatics,* 2009, vol. 25 (14), 1754-1760
- **LI, H. et al.** Fast and accurate long-read alignment with Burrows-Wheeler transform. *Bioinformatics,* 2010, vol. 26 (5), 589-595
- **LI, HENG et al.** The Sequence Alignment / Map format and SAMtools. *Bioinformatics,* 2009, vol. 25 (16), 2078-2079
- **QUINLAN, A. R. et al.** BEDtools: a flexible suite of utilities for comparing genomic features. *Bioinformatics,* 2010, vol. 26 (6), 841-842
- **ALTSCHUL, S. A. et al.** Basic Local Alignment Search Tool. *Journal of Molecular Biology,* October 1990, vol. 215, 403-410
- Improved tools for biological sequence comparison. **PEARSON, W. R. et al.** Proceedings of the National Academy of Sciences of the United States of America. National Academy of Sciences, April 1988, vol. 85, 2444-2448

SEQUENCE LISTING

<110> FUJI FILM CORPORATION

<120> METHOD FOR QUANTIFICATION OF CHROMOSOME NUMBER VARIATION

<130> W-5931PCT

<150> JP2016-033284
<151> 2016-02-24

<160> 107

<170> PatentIn version 3.5

<210> 1
<211> 37
<212> DNA
<213> Artificial Sequence

<220>
<223> chr13: 20763380: Fwd

<400> 1
cgctcttccg atctctgctt cgatgcggac cttctgg 37

<210> 2
<211> 37
<212> DNA
<213> Artificial Sequence

<220>
<223> chr13: 20763380: Rev

<400> 2
cgctcttccg atctgactct cccacatccg gctatgg 37

<210> 3
<211> 37
<212> DNA

<213> Artificial Sequence

<220>
<223> chr13: 21205192: Fwd

<400> 3
cgctcttccg atctctgttt ccccgaccat aagcttg 37

<210> 4
<211> 37
<212> DNA
<213> Artificial Sequence

<220>
<223> chr13: 21205192: Rev

<400> 4
cgctcttccg atctgacata cagggctgag agattgg 37

<210> 5
<211> 37
<212> DNA
<213> Artificial Sequence

<220>
<223> chr13: 21620085: Fwd

<400> 5
cgctcttccg atctctgtga taaggtccga actttgg 37

<210> 6
<211> 37
<212> DNA
<213> Artificial Sequence

<220>
<223> chr13: 21620085: Rev

<400> 6
cgctcttccg atctgacgcg actgcaagag attcgtg 37

<210> 7
<211> 37
<212> DNA
<213> Artificial Sequence

<220>
<223> chr13: 23898509: Fwd

<400> 7
cgctcttccg atctctgatt tgctgctgac cagggtg 37

<210> 8
<211> 37
<212> DNA
<213> Artificial Sequence

<220>

<223> chr13: 23898509: Rev

<400> 8
cgctcttccg atctgacagg tacagcttcc catctgg 37

<210> 9
<211> 37
<212> DNA
<213> Artificial Sequence

<220>
<223> chr13: 24797913: Fwd

<400> 9
cgctcttccg atctctgccg tgtgtgagat tctcgtg 37

<210> 10
<211> 37
<212> DNA
<213> Artificial Sequence

<220>
<223> chr13: 24797913: Rev

<400> 10
cgctcttccg atctgacact gctcagggtc ctctgtg 37

<210> 11
<211> 37
<212> DNA
<213> Artificial Sequence

<220>
<223> chr13: 25009099: Fwd

<400> 11
cgctcttccg atctctggta aagcctccag gatgttg 37

<210> 12
<211> 37
<212> DNA
<213> Artificial Sequence

<220>
<223> chr13: 25009099: Rev

<400> 12
cgctcttccg atctgacctg gcacttgtgc tgactgg 37

<210> 13
<211> 37
<212> DNA
<213> Artificial Sequence

<220>
<223> chr13: 25029218: Fwd

<400> 13

cgctcttccg atctctgcca aagcgcactc acctgtg 37

<210> 14
<211> 37
<212> DNA
<213> Artificial Sequence

<220>
<223> chr13: 25029218: Rev

<400> 14
cgctcttccg atctgactag ccagtgagag cgaagtg 37

<210> 15
<211> 37
<212> DNA
<213> Artificial Sequence

<220>
<223> chr13: 25265103: Fwd

<400> 15
cgctcttccg atctctgggc ctagaggacg atgcttg 37

<210> 16
<211> 37
<212> DNA
<213> Artificial Sequence

<220>
<223> chr13: 25265103: Rev

<400> 16
cgctcttccg atctgactgt tgataaccat gccggtg 37

<210> 17
<211> 37
<212> DNA
<213> Artificial Sequence

<220>
<223> chr13: 25266932: Fwd

<400> 17
cgctcttccg atctctgtgc tggacagtga ctcatgg 37

<210> 18
<211> 37
<212> DNA
<213> Artificial Sequence

<220>
<223> chr13: 25266932: Rev

<400> 18
cgctcttccg atctgaccat tttcctgtcc tggcttg 37

<210> 19

<211> 37
<212> DNA
<213> Artificial Sequence

<220>
<223> chr13: 25453420: Fwd

<400> 19
cgctcttccg atctctgatc cagttcatat gccgttg 37

<210> 20
<211> 37
<212> DNA
<213> Artificial Sequence

<220>
<223> chr13: 25453420: Rev

<400> 20
cgctcttccg atctgacgcg ttgctgtcat tcctttg 37

<210> 21
<211> 37
<212> DNA
<213> Artificial Sequence

<220>
<223> chr18: 346821: Fwd

<400> 21
cgctcttccg atctctgagg ttctgctcgt tggcttg 37

<210> 22
<211> 37
<212> DNA
<213> Artificial Sequence

<220>
<223> chr18: 346821: Rev

<400> 22
cgctcttccg atctgaccca agaaggacac ggattgg 37

<210> 23
<211> 37
<212> DNA
<213> Artificial Sequence

<220>
<223> chr18: 3075712: Fwd

<400> 23
cgctcttccg atctctgagc ttcccgggaa attagtg 37

<210> 24
<211> 37
<212> DNA
<213> Artificial Sequence

<220>
<223> chr18: 3075712: Rev

<400> 24
cgctcttccg atctgacgaa ttttaatcgc ccctgtg 37

<210> 25
<211> 37
<212> DNA
<213> Artificial Sequence

<220>
<223> chr18: 3188976: Fwd

<400> 25
cgctcttccg atctctggga ctgcttagat gccgtgg 37

<210> 26
<211> 37
<212> DNA
<213> Artificial Sequence

<220>
<223> chr18: 3188976: Rev

<400> 26
cgctcttccg atctgacagt caaaagcatg tcagtgg 37

<210> 27
<211> 37
<212> DNA
<213> Artificial Sequence

<220>
<223> chr18: 3457539: Fwd

<400> 27
cgctcttccg atctctgctc tgtggagtcc gtgatgg 37

<210> 28
<211> 37
<212> DNA
<213> Artificial Sequence

<220>
<223> chr18: 3457539: Rev

<400> 28
cgctcttccg atctgacatg cagtcacagt ggtatgg 37

<210> 29
<211> 37
<212> DNA
<213> Artificial Sequence

<220>
<223> chr18: 5416160: Fwd

<400> 29
cgctcttccg atctctgggt aatgctgcaa gctctgg 37

<210> 30
<211> 37
<212> DNA
<213> Artificial Sequence

<220>
<223> chr18: 5416160: Rev

<400> 30
cgctcttccg atctgaccct aggggatcaa gatgtgg 37

<210> 31
<211> 37
<212> DNA
<213> Artificial Sequence

<220>
<223> chr18: 5956238: Fwd

<400> 31
cgctcttccg atctctgatt catcccctga cttcttg 37

<210> 32
<211> 37
<212> DNA
<213> Artificial Sequence

<220>
<223> chr18: 5956238: Rev

<400> 32
cgctcttccg atctgactat ttgcagcaga tcgatgg 37

<210> 33
<211> 37
<212> DNA
<213> Artificial Sequence

<220>
<223> chr18: 6943264: Fwd

<400> 33
cgctcttccg atctctgcac caacataaat gggattg 37

<210> 34
<211> 37
<212> DNA
<213> Artificial Sequence

<220>
<223> chr18: 6943264: Rev

<400> 34
cgctcttccg atctgacgcc actgtgctct gtgatgg 37

<210> 35
<211> 37
<212> DNA
<213> Artificial Sequence

<220>
<223> chr18: 6983194: Fwd

<400> 35
cgctcttccg atctctgagt cctgtgagca tctctgg 37

<210> 36
<211> 37
<212> DNA
<213> Artificial Sequence

<220>
<223> chr18: 6983194: Rev

<400> 36
cgctcttccg atctgactga gtgaattggc aagtttg 37

<210> 37
<211> 37
<212> DNA
<213> Artificial Sequence

<220>
<223> chr18: 8796223: Fwd

<400> 37
cgctcttccg atctctggca gtttgcaggg actcttg 37

<210> 38
<211> 37
<212> DNA
<213> Artificial Sequence

<220>
<223> chr18: 8796223: Rev

<400> 38
cgctcttccg atctgaccac tccaggttcg ctcatgg 37

<210> 39
<211> 37
<212> DNA
<213> Artificial Sequence

<220>
<223> chr18: 8798185: Fwd

<400> 39
cgctcttccg atctctgctc ctgcctgttt cagggtg 37

<210> 40
<211> 37
<212> DNA

<213> Artificial Sequence

<220>
<223> chr18: 8798185: Rev

<400> 40
cgctcttccg atctgacaat ctgcacccgg gagtgtg 37

<210> 41
<211> 37
<212> DNA
<213> Artificial Sequence

<220>
<223> chr21: 16340289: Fwd

<400> 41
cgctcttccg atctctggca acagtctggc ttttttg 37

<210> 42
<211> 37
<212> DNA
<213> Artificial Sequence

<220>
<223> chr21: 16340289: Rev

<400> 42
cgctcttccg atctgacggg atcaggtact gccgttg 37

<210> 43
<211> 37
<212> DNA
<213> Artificial Sequence

<220>
<223> chr21: 28212760: Fwd

<400> 43
cgctcttccg atctctgcaa ggtgctacat gtgctgg 37

<210> 44
<211> 37
<212> DNA
<213> Artificial Sequence

<220>
<223> chr21: 28212760: Rev

<400> 44
cgctcttccg atctgaccct ttgcagggga atgtttg 37

<210> 45
<211> 37
<212> DNA
<213> Artificial Sequence

<220>

<223> chr21: 28305212: Fwd

<400> 45
cgctcttccg atctctggag cagcgtacca ttgggtg 37

<210> 46
<211> 37
<212> DNA
<213> Artificial Sequence

<220>
<223> chr21: 28305212: Rev

<400> 46
cgctcttccg atctgaccag tgttctcgct catgtgg 37

<210> 47
<211> 37
<212> DNA
<213> Artificial Sequence

<220>
<223> chr21: 30408670: Fwd

<400> 47
cgctcttccg atctctgtgt ctcccccttt ttagttg 37

<210> 48
<211> 37
<212> DNA
<213> Artificial Sequence

<220>
<223> chr21: 30408670: Rev

<400> 48
cgctcttccg atctgacttt acctggcttt ggagttg 37

<210> 49
<211> 37
<212> DNA
<213> Artificial Sequence

<220>
<223> chr21: 30464866: Fwd

<400> 49
cgctcttccg atctctggtc agcaagttgg ctactgg 37

<210> 50
<211> 37
<212> DNA
<213> Artificial Sequence

<220>
<223> chr21: 30464866: Rev

<400> 50

cgctcttccg atctgacagc cttaggctcc catggtg 37

<210> 51
<211> 37
<212> DNA
<213> Artificial Sequence

<220>
<223> chr21: 31709691: Fwd

<400> 51
cgctcttccg atctctgtgc tgagtgcttt ctgattg 37

<210> 52
<211> 37
<212> DNA
<213> Artificial Sequence

<220>
<223> chr21: 31709691: Rev

<400> 52
cgctcttccg atctgactca cagacgatag ctgtgtg 37

<210> 53
<211> 37
<212> DNA
<213> Artificial Sequence

<220>
<223> chr21: 31812772: Fwd

<400> 53
cgctcttccg atctctgctt ctcctcctgc tgttttg 37

<210> 54
<211> 37
<212> DNA
<213> Artificial Sequence

<220>
<223> chr21: 31812772: Rev

<400> 54
cgctcttccg atctgaccca aagtgcagga tgtctgg 37

<210> 55
<211> 37
<212> DNA
<213> Artificial Sequence

<220>
<223> chr21: 32253513: Fwd

<400> 55
cgctcttccg atctctggag actcctccca ctggttg 37

<210> 56

<211> 37
<212> DNA
<213> Artificial Sequence

<220>
<223> chr21: 32253513: Rev

<400> 56
cgctcttccg atctgacaac ccttgccagg tgacttg 37

<210> 57
<211> 37
<212> DNA
<213> Artificial Sequence

<220>
<223> chr21: 32638549: Fwd

<400> 57
cgctcttccg atctctgtct tcagcagcag ctggtgg 37

<210> 58
<211> 37
<212> DNA
<213> Artificial Sequence

<220>
<223> chr21: 32638549: Rev

<400> 58
cgctcttccg atctgaccca attccttggg tgacttg 37

<210> 59
<211> 37
<212> DNA
<213> Artificial Sequence

<220>
<223> chr21: 33694120: Fwd

<400> 59
cgctcttccg atctctggcg gaacccatgt acctgtg 37

<210> 60
<211> 37
<212> DNA
<213> Artificial Sequence

<220>
<223> chr21: 33694120: Rev

<400> 60
cgctcttccg atctgacaaa cagagcagaa gtgggtg 37

<210> 61
<211> 37
<212> DNA
<213> Artificial Sequence

<220>
<223> chrX: 2779570: Fwd

<400> 61
cgctcttccg atctctgaga ctcactccac gtgtgtg 37

<210> 62
<211> 37
<212> DNA
<213> Artificial Sequence

<220>
<223> chrX: 2779570: Rev

<400> 62
cgctcttccg atctgacaga acccagtggt gaatttg 37

<210> 63
<211> 37
<212> DNA
<213> Artificial Sequence

<220>
<223> chrX: 2951434: Fwd

<400> 63
cgctcttccg atctctgctt ccccttctgt gggtgtg 37

<210> 64
<211> 37
<212> DNA
<213> Artificial Sequence

<220>
<223> chrX: 2951434: Rev

<400> 64
cgctcttccg atctgacagg ataaaacaat gggttgg 37

<210> 65
<211> 37
<212> DNA
<213> Artificial Sequence

<220>
<223> chrX: 3241050: Fwd

<400> 65
cgctcttccg atctctgctg ttgccgtctc ttcatgg 37

<210> 66
<211> 37
<212> DNA
<213> Artificial Sequence

<220>
<223> chrX: 3241050: Rev

<400> 66
cgctcttccg atctgacacc tctggaggaa gttgttg 37

<210> 67
<211> 37
<212> DNA
<213> Artificial Sequence

<220>
<223> chrX: 6995417: Fwd

<400> 67
cgctcttccg atctctggaa ctccttgtgg cggcttg 37

<210> 68
<211> 37
<212> DNA
<213> Artificial Sequence

<220>
<223> chrX: 6995417: Rev

<400> 68
cgctcttccg atctgaccct gcaagaaggt cttatgg 37

<210> 69
<211> 37
<212> DNA
<213> Artificial Sequence

<220>
<223> chrX: 14027177: Fwd

<400> 69
cgctcttccg atctctgctc catggcttgg atcttgg 37

<210> 70
<211> 37
<212> DNA
<213> Artificial Sequence

<220>
<223> chrX: 14027177: Rev

<400> 70
cgctcttccg atctgacagc gcctggacag ctatgtg 37

<210> 71
<211> 37
<212> DNA
<213> Artificial Sequence

<220>
<223> chrX: 16168467: Fwd

<400> 71
cgctcttccg atctctgtac gccaggtgtc tcgcttg 37

<210> 72
<211> 37
<212> DNA
<213> Artificial Sequence

<220>
<223> chrX: 16168467: Rev

<400> 72
cgctcttccg atctgactcc agataaaggc ggctttg 37

<210> 73
<211> 37
<212> DNA
<213> Artificial Sequence

<220>
<223> chrX: 16627756: Fwd

<400> 73
cgctcttccg atctctggac agtttgcaac cctgttg 37

<210> 74
<211> 37
<212> DNA
<213> Artificial Sequence

<220>
<223> chrX: 16627756: Rev

<400> 74
cgctcttccg atctgactct gctttaatcg catcgtg 37

<210> 75
<211> 37
<212> DNA
<213> Artificial Sequence

<220>
<223> chrX: 17746244: Fwd

<400> 75
cgctcttccg atctctgtgc aggtctggag gaagttg 37

<210> 76
<211> 37
<212> DNA
<213> Artificial Sequence

<220>
<223> chrX: 17746244: Rev

<400> 76
cgctcttccg atctgactac caggcacttt gtcatgg 37

<210> 77
<211> 37
<212> DNA

<213> Artificial Sequence

<220>
<223> chrX: 19375782: Fwd

<400> 77
cgctcttccg atctctgtaa taaagggcct gcgtttg 37

<210> 78
<211> 37
<212> DNA
<213> Artificial Sequence

<220>
<223> chrX: 19375782: Rev

<400> 78
cgctcttccg atctgaccac tcatactgtg tccgtgg 37

<210> 79
<211> 37
<212> DNA
<213> Artificial Sequence

<220>
<223> chrX: 22291606: Fwd

<400> 79
cgctcttccg atctctgagt taccagcgct tcgcttg 37

<210> 80
<211> 37
<212> DNA
<213> Artificial Sequence

<220>
<223> chrX: 22291606: Rev

<400> 80
cgctcttccg atctgacatg ttgcacagac ggtagtg 37

<210> 81
<211> 37
<212> DNA
<213> Artificial Sequence

<220>
<223> chrY: 2710802: Fwd

<400> 81
cgctcttccg atctctgaac aagggcaaga agttgtg 37

<210> 82
<211> 37
<212> DNA
<213> Artificial Sequence

<220>

<223> chrY: 2710802: Rev

<400> 82
cgctcttccg atctgaccac catcaatgtg gaaattg 37

<210> 83
<211> 37
<212> DNA
<213> Artificial Sequence

<220>
<223> chrY: 6740540: Fwd

<400> 83
cgctcttccg atctctgctt cagcacagat gttttgg 37

<210> 84
<211> 37
<212> DNA
<213> Artificial Sequence

<220>
<223> chrY: 6740540: Rev

<400> 84
cgctcttccg atctgacttt tgtttgcctg ccttgtg 37

<210> 85
<211> 37
<212> DNA
<213> Artificial Sequence

<220>
<223> chrY: 7262370: Fwd

<400> 85
cgctcttccg atctctgtgt catcaatagt tggctgg 37

<210> 86
<211> 37
<212> DNA
<213> Artificial Sequence

<220>
<223> chrY: 7262370: Rev

<400> 86
cgctcttccg atctgacagt tcccttttgt agggtgg 37

<210> 87
<211> 37
<212> DNA
<213> Artificial Sequence

<220>
<223> chrY: 8624945: Fwd

<400> 87

cgctcttccg atctctgttg ctgtgtgaag tccttgg 37

<210> 88
<211> 37
<212> DNA
<213> Artificial Sequence

<220>
<223> chrY: 8624945: Rev

<400> 88
cgctcttccg atctgacaag cggacagctg tgtctgg 37

<210> 89
<211> 37
<212> DNA
<213> Artificial Sequence

<220>
<223> chrY: 8632095: Fwd

<400> 89
cgctcttccg atctctgcct gggagctcgt gagtttg 37

<210> 90
<211> 37
<212> DNA
<213> Artificial Sequence

<220>
<223> chrY: 8632095: Rev

<400> 90
cgctcttccg atctgactca cgtctgccta gattttg 37

<210> 91
<211> 37
<212> DNA
<213> Artificial Sequence

<220>
<223> chrY: 14655058: Fwd

<400> 91
cgctcttccg atctctgcag agtatcaggc cttctgg 37

<210> 92
<211> 37
<212> DNA
<213> Artificial Sequence

<220>
<223> chrY: 14655058: Rev

<400> 92
cgctcttccg atctgacggc ttaccagctt gtagtgg 37

<210> 93

<211> 37
<212> DNA
<213> Artificial Sequence

<220>
<223> chrY: 14756402: Fwd

<400> 93
cgctcttccg atctctgcca accatcacga aaattgg 37

<210> 94
<211> 37
<212> DNA
<213> Artificial Sequence

<220>
<223> chrY: 14756402: Rev

<400> 94
cgctcttccg atctgactcg tctcgtactg gagattg 37

<210> 95
<211> 37
<212> DNA
<213> Artificial Sequence

<220>
<223> chrY: 14944834: Fwd

<400> 95
cgctcttccg atctctgtga tactccaatt gtggtgg 37

<210> 96
<211> 37
<212> DNA
<213> Artificial Sequence

<220>
<223> chrY: 14944834: Rev

<400> 96
cgctcttccg atctgacctg tgtttttctt tgcggtg 37

<210> 97
<211> 37
<212> DNA
<213> Artificial Sequence

<220>
<223> chrY: 15872674: Fwd

<400> 97
cgctcttccg atctctgccc cagtggacag agttttg 37

<210> 98
<211> 37
<212> DNA
<213> Artificial Sequence

<220>
<223> chrY: 15872674: Rev

<400> 98
cgctcttccg atctgacgga gccaatgctg tgatgtg 37

<210> 99
<211> 37
<212> DNA
<213> Artificial Sequence

<220>
<223> chrY: 22902924: Fwd

<400> 99
cgctcttccg atctctgtga cattgaaggt agcgttg 37

<210> 100
<211> 37
<212> DNA
<213> Artificial Sequence

<220>
<223> chrY: 22902924: Rev

<400> 100
cgctcttccg atctgacgag aaatcggagt tcattgg 37

<210> 101
<211> 69
<212> DNA
<213> Artificial Sequence

<220>
<223> P5 sequence

<400> 101

**aatgatacgg cgaccaccga gatctacact atagccttct ttccctacac gacgctcttc 60**

**cgatctctg 69**

<210> 102
<211> 69
<212> DNA
<213> Artificial Sequence

<220>
<223> P7 sequence-1

<400> 102

**caagcagaag acggcatacg agatcgagta atgtgactgg agttcagacg tgtgctcttc 60**

**cgatctgac 69**

<210> 103

<211> 69
<212> DNA
<213> Artificial Sequence

<220>
<223> P7 sequence-2

<400> 103

**caagcagaag acggcatacg agattctccg gagtgactgg agttcagacg tgtgctcttc 60**

**cgatctgac 69**

<210> 104
<211> 69
<212> DNA
<213> Artificial Sequence

<220>
<223> P7 sequence-3

<400> 104

**caagcagaag acggcatacg agataatgag cggtgactgg agttcagacg tgtgctcttc 60**

**cgatctgac 69**

<210> 105
<211> 69
<212> DNA
<213> Artificial Sequence

<220>
<223> P7 sequence-4

<400> 105

**caagcagaag acggcatacg agatggaatc tcgtgactgg agttcagacg tgtgctcttc 60**

**cgatctgac 69**

<210> 106
<211> 69
<212> DNA
<213> Artificial Sequence

<220>
<223> P7 sequence-5

<400> 106

**caagcagaag acggcatacg agatttctga atgtgactgg agttcagacg tgtgctcttc 60**

**cgatctgac 69**

<210> 107
<211> 69
<212> DNA
<213> Artificial Sequence

<220>
<223> P7 sequence-6

<400> 107

```
caagcagaag acggcatacg agatacgaat tcgtgactgg agttcagacg tgtgctcttc      60

cgatctgac                                                              69
```